# EUROPEAN PATENT APPLICATION

(11) **EP 4 019 051 A1**
(43) Date of publication of application: **29.06.2022**
(21) Application number: 20216739.1
(22) Date of filing: 22.12.2020
(51) Int. Cl.: A61K 51/08, A61K 51/04, A61K 103/30, A61K 103/40, C07D 471/08

(54) **BISPIDINE DERIVATIVES AND THE USE THEREOF**

(71) Applicant: Helmholtz-Zentrum Dresden - Rossendorf e.V., 01328 Dresden (DE); Universität Heidelberg, 69117 Heidelberg (DE)
(72) Inventor: Comba, Peter, 69257 Wiesenbach (DE); Cieslik, Patrick, 69221 Dossenheim (DE); Kubeil, Manja, 01328 Dresden (DE); Stephan, Holger, 01328 Dresden (DE)
(74) Representative: Riechelmann & Carlsohn Patentanwälte PartG mbB

(57) **Abstract**

The invention relates to a compound of general formula I 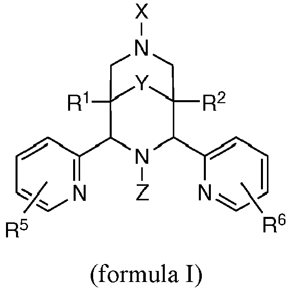 in which
X is selected from consisting of a first group, a second group or a third group, wherein the first group consists of 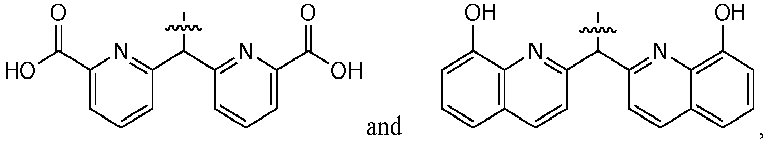 the second group consists of 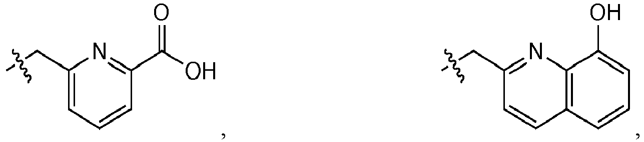 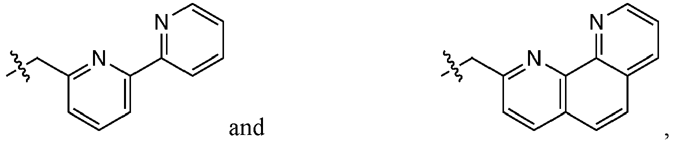 and the third group consists of 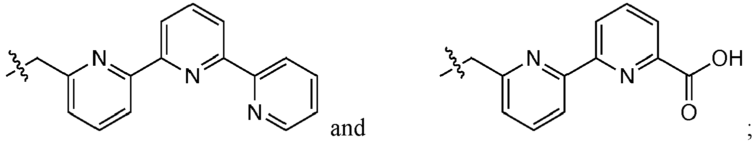 Y is a group of general formula II or of general formula III 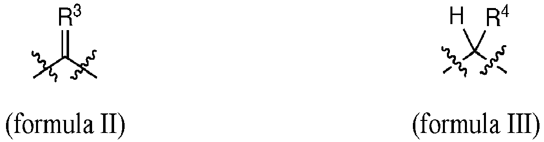 if X is selected from the first group, Z is selected from a first group consisting of 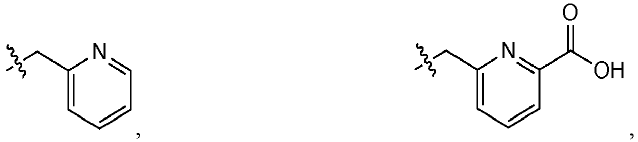 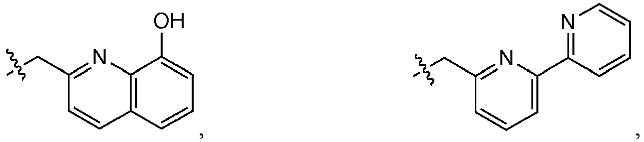 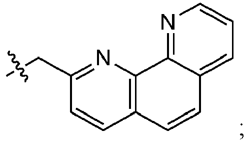 if X is selected from the second group, Z is selected from a second group consisting of 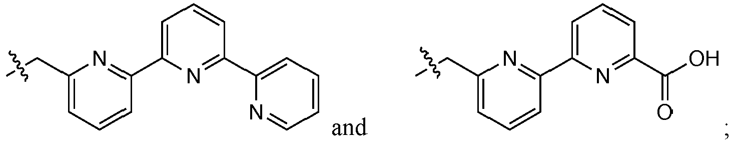 if X is selected from the third group, Z is selected from a third group consisting of 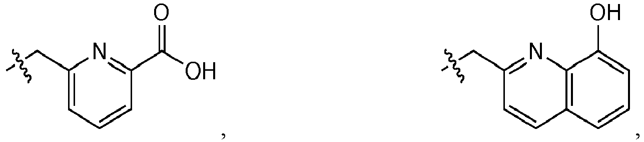 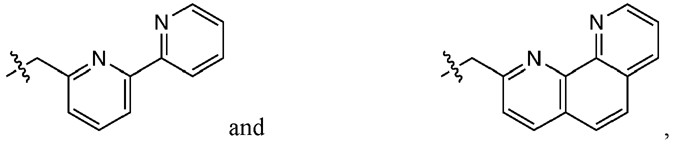 R¹ and R² independently are selected from the group consisting of a substituted or unsubstituted C₁-C₆ alkyl group, a substituted or unsubstituted C₁-C₆ heteroalkyl group, -C(O)-O-R^{a}, -C(O)-NR^{b}R^{c}, -C(O)-C(R^{a})₂-NR^{b}R^{c}, and -C(O)-NR^{b}-(CH₂)ₙ-C(O)-O-R^{a}, wherein R^{a}, R^{b} and R^{c} each independently are selected from the group consisting of hydrogen, a substituted or unsubstituted C₁-C₆ alkyl group, a substituted or unsubstituted C₁-C₆ heteroalkyl group, a substituted or unsubstituted aryl group and a group L and n is an integer from 1 to 10;
R³ is selected from the group consisting of oxygen, sulfur, =NR^{d}, and =CHR^{d}, wherein R^{d} is selected from the group consisting of hydrogen, a substituted or unsubstituted C₁-C₆ alkyl group, a substituted or unsubstituted C₁-C₆ heteroalkyl group, a substituted or unsubstituted aryl group, -O-R^{e}, -C(O)-O-R^{e}, -C(O)-NR^{f}R^{g}, and a group L, R^{e} is hydrogen or a substituted or unsubstituted C₁-C₆ alkyl group, and R^{f} and R^{g} each independently are hydrogen or a substituted or unsubstituted C₁-C₆ alkyl group; and
R⁴ is selected from the group consisting of -OR^{h}, -SR^{h}, -NHR^{h} and -CH₂R^{h}, wherein R^{h} is selected from the group consisting of hydrogen, a substituted or unsubstituted C₁-C₆ alkyl group, a substituted or unsubstituted C₁-C₆ alkenyl group, a substituted or unsubstituted C₁-C₆ alkynyl group, a substituted or unsubstituted C₁-C₆ heteroalkyl group, a substituted or unsubstituted aryl group, -C(O)-(CH₂)ₘ-R^{k}, -C(O)-(CH₂)ₘ-NR^{m}Rⁿ, a group -A-L and a group L, R^{k} is selected from the group consisting of a substituted or unsubstituted C₁-C₆ alkyl group, a substituted or unsubstituted C₁-C₆ heteroalkyl group, a substituted or unsubstituted aryl group, and a substituted or unsubstituted carboxy group, R^{m} and Rⁿ each independently are hydrogen or a substituted or unsubstituted C₁-C₆ alkyl group, and m is 0 or an integer from 1 to 10;
R⁵ and R⁶ independently are selected from the group consisting of hydrogen, chloro, bromo, iodo, and O-R^{o}, wherein R° is a substituted or unsubstituted C₁-C₆ alkyl group;
A is a linker group and
L is an amino acid residue or a peptide.

## Description

The invention relates to bispidine derivatives and the use thereof for the complexation of metal ions. Moreover, it relates to a method for preparing complexes having such bispidine derivatives as ligands, as well as such complexes.

Nuclear medicine is considered to be a fast growing, interdisciplinary field of research, which makes use of open radioactive nuclides for diagnosis as well as therapy purposes. These so-called radiopharmaceuticals offer a new, more advantageous approach for cancer diagnosis and treatment. The biggest part of nuclear-medically usable radio-nuclides are metals, which is why the focus research in modern radiopharmaceuticals is just on these ("Therapeutic Radiopharmaceuticals" Chem. Rev. 1999, 99, 9, 2269-2292; "Radiopharmaceutical therapy in cancer: clinical advances and challenges" Nat. Rev. Drug. Discov. 2020, 19 (9), 589-608).

In this regard, the radio-active nuclides of the lanthanoids and actinides take a special position, in particular Lutetium-177 as beta-emitting radionuclide and Actinium-225 as alpha-emitting radionuclide. Due to their long half-lives (¹⁷⁷Lu: 6,7 d; ²²⁵Ac: 10 d) they are ideally suitable for use in radio therapy. Lutetium-177 preparations are already used as such ("Lutetium Lu-177 Dotatate Approved by FDA", Cancer Discovery 2018, 8, 4, OF2; Lutathera^{®}: "The first FDA- and EMA-approved radiopharmaceutical for peptide receptor radionuclide therapy", Pharmaceuticals 2019, 12, 114). Development of new ligands for Lutetium-177 as well as Actinium-225 is a current question in research, since common ligands of these metal nuclides, for example 1,4,7,10-tetraazacyclododecane-1,4,7,10-tetraacetic acid (DOTA) and 2-[Bis[2-[bis(carboxymethyl)amino]ethyl]amino]acetic acid (DTPA), in spite of their high thermodynamic stability have drawbacks that have to be eliminated. Successful radiolabelling with DOTA ligands can only be realized by high temperatures, which is why this ligand for many applications becomes unsuitable. Alternatively, DTPA and its derivatives are used, which, however, are kinetic inert only to a low degree. ("Radioactive Main Group and Rare Earth Metals for Imaging and Therapy", Chem. Rev. 2019, 119, 2, 902-956).

Thus, each of the known ligands is associated with disadvantages. DOTA requires high temperatures of 80°C to be able to carry out a complete radiolabelling. However, antibodies and target vectors often are thermolabile. Certainly, DTPA allows a fast complexation, but its kinetic lability is proved. In vivo decomplexation releases radioactive metal. Macropa (1,7,10,16-tetraoxa-4,13-diazacyclooctadecane) and its derivatives are used as complexing agents for Targeted Alpha Therapy (TAT), mainly for actinium-225. However, only an insufficient conversion of the radiolabeling with lanthanides, such as Lu³⁺ is achieved, the specific activity is low and the complexes are kinetically labile, so that radioactive metal ions can be liberated ("Implementing f-Block Metal ions in Medicine: Tuning Size Selectivity of expanded Macrocycles", Inorg. Chem. 2019, 58, 10483.).

Bispidine derivatives are known from the prior art that are used as ligands for radiolabelings. Bispidine is a compound of formula PI 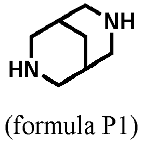 These bispidine derivatives are H₂bispa² and H₂bispox² ("Octadentate Picolinic Acid-Based Bispidine Ligand for Radiometal Ions" Chem. Eur. J. 2017, 23, 15945 - 15956; "Octadentate Oxine-Armed Bispidine Ligand for Radiopharmaceutical Chemistry" Inorg. Chem. 2019, 58, 8685-8693.). Bispidine derivatives are also used as ligands for ⁶⁴Cu, which is a radiometal used in PET imaging techniques (Comba, P.; Kerscher, M.; Ruck, K. & Starke, M. Bispidines for radiopharmaceuticals Dalton Transactions, 2018, 47, 9202-9220;Singh, G.; Zarschler, K.; Hunoldt, S.; Martinez, I. I. S.; Ruehl, C. L.; Matterna, M.; Bergmann, R.; Mathe, D.; Hegedus, N.; Bachmann, M.; Comba, P. & Stephan, H. Versatile Bispidine-Based Bifunctional Chelators for (64) Cu(II) -Labelling of Biomolecules Chemistry - A European Journal, 2020, 26, 1989-2001.)

The problem of the invention is to eliminate the drawbacks according to the prior art. In particular, there are provided compounds that are suitable as ligands for the complexation of large metal ions under mild conditions. In addition, there are provided complexes having a high kinetic stability. Finally, there are provided the use of the compounds for the complexation of metal ions and a method for preparing the complexes.

This problem is solved by the features of claims 1, 10, 12, and 14. Practical developments of the inventions result from the features of the dependent claims.

According to the invention there is provided a compound of general formula I 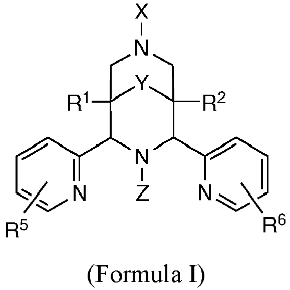 in which
X is selected from a first group or a second group or a third group, wherein the first group consists of 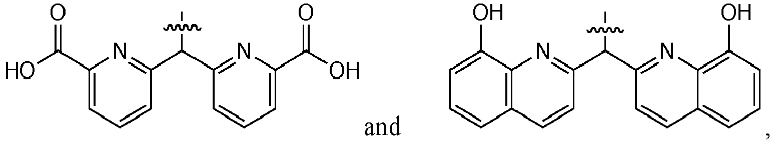 the second group consists of 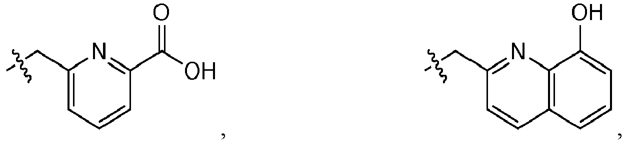 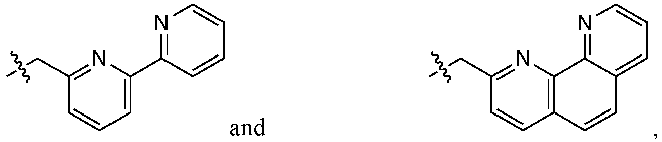 and the third group consists of 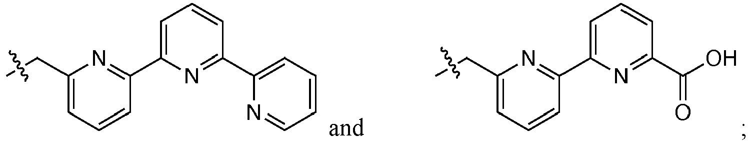
Y is a group of general formula II or of general formula III 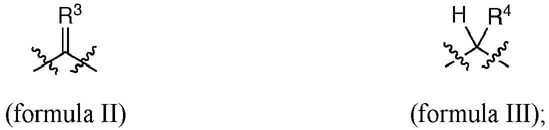
if X is selected from the first group, Z is selected from a first group consisting of 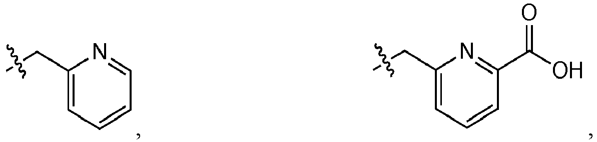 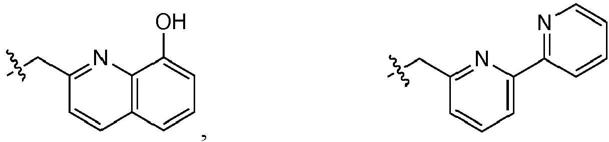 and 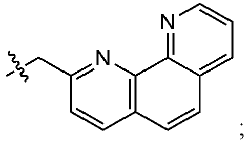
if X is selected from the second group, Z is selected from a second group consisting of 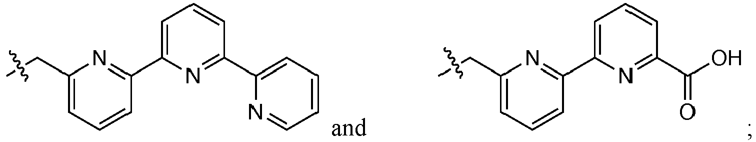
if X is selected from the third group, Z is selected from a third group consisting of 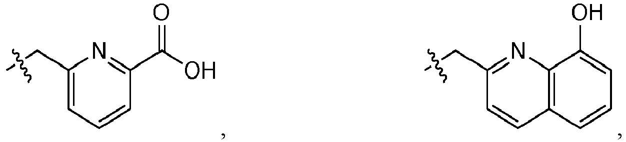 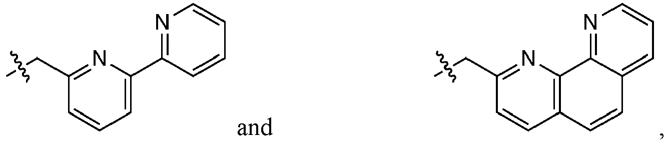
R¹ and R² independently are selected from the group consisting of a substituted or unsubstituted C₁-C₆ alkyl group, a substituted or unsubstituted C₁-C₆ heteroalkyl group, -C(O)-O-R^{a}, -C(O)-NR^{b}R^{c}, -C(O)-C(R^{a})₂-NR^{b}R^{c}, and -C(O)-NR^{b}-(CH₂)ₙ-C(O)-O-R^{a}, wherein R^{a}, R^{b} and R^{c} each independently are selected from the group consisting of hydrogen, a substituted or unsubstituted C₁-C₆ alkyl group, a substituted or unsubstituted C₁-C₆ heteroalkyl group, a substituted or unsubstituted aryl group, a group -A-L, and a group L and n is an integer from 1 to 10;
R³ is selected from the group consisting of oxygen, sulfur, =NR^{d}, and =CHR^{d}, wherein R^{d} is selected from the group consisting of hydrogen, a substituted or unsubstituted C₁-C₆ alkyl group, a substituted or unsubstituted C₁-C₆ heteroalkyl group, a substituted or unsubstituted aryl group, -O-R^{e}, -C(O)-O-R^{e}, -C(O)-NR^{f}R^{g}, a group -A-L, and a group L, R^{e} is hydrogen or a substituted or unsubstituted C₁-C₆ alkyl group, and R^{f} and R^{g} each independently are hydrogen or a substituted or unsubstituted C₁-C₆ alkyl group; and
R⁴ is selected from the group consisting of -OR^{h}, -SR^{h}, -NHR^{h} and -CH₂R^{h}, wherein R^{h} is selected from the group consisting of hydrogen, a substituted or unsubstituted C₁-C₆ alkyl group, a substituted or unsubstituted C₁-C₆ alkenyl group, a substituted or unsubstituted C₁-C₆ alkynyl group, a substituted or unsubstituted C₁-C₆ heteroalkyl group, a substituted or unsubstituted aryl group, -C(O)-(CH₂)ₘ-R^{k}, -C(O)-(CH₂)ₘ-NR^{m}Rⁿ, a group -A-L, and a group L, R^{k} is selected from the group consisting of a substituted or unsubstituted C₁-C₆ alkyl group, a substituted or unsubstituted C₁-C₆ heteroalkyl group, a substituted or unsubstituted aryl group, and a substituted or unsubstituted carboxy group, R^{m} and Rⁿ each independently are hydrogen or a substituted or unsubstituted C₁-C₆ alkyl group, and m is 0 or an integer from 1 to 10;
R⁵ and R⁶ independently are selected from the group consisting of hydrogen, chloro, bromo, iodo, and O-R^{o}, wherein R^{o} is a substituted or unsubstituted C₁-C₆ alkyl group;
A is a linker group; and
L is selected from the group consisting of an amino acid residue and a peptide.

In a compound of formula I it may be provided that X, Y, and Z have the meanings given above, wherein
R¹ and R² independently are selected from the group consisting of a substituted or unsubstituted C₁-C₆ alkyl group, -C(O)-O-R^{a}, -C(O)-NR^{b}R^{c}, -C(O)-C(R^{a})₂-NR^{b}R^{c}, and -C(O)-NR^{b}-(CH₂)ₙ-C(O)-O-R^{a}, wherein R^{a}, R^{b}, and R^{c} each independently are selected from the group consisting of hydrogen, a substituted or unsubstituted C₁-C₆ alkyl group, a substituted or unsubstituted aryl group, a group -A-L, and a group L; and n is an integer from 1 to 10;
R³ is selected from the group consisting of oxygen, sulfur, =NR^{d}, and =CHR^{d}, wherein R^{d} is selected from the group consisting of hydrogen, a substituted or unsubstituted C₁-C₆ alkyl group, a substituted or unsubstituted aryl group, -O-R^{e}, - C(O)-O-R^{e}, -C(O)-NR^{f}R^{g}, a group -A-L, and a group L, R^{e} is hydrogen or a substituted or unsubstituted C₁-C₆ alkyl group, and R^{f} and R^{g} each independently are hydrogen or a substituted or unsubstituted C₁-C₆ alkyl group; and
R⁴ is selected from the group consisting of -OR^{h}, -SR^{h}, -NHR^{h}, and -CH₂R^{h}, wherein R^{h} is selected from the group consisting of hydrogen, a substituted or unsubstituted C₁-C₆ alkyl group, a substituted or unsubstituted C₁-C₆ alkenyl group, a substituted or unsubstituted C₁-C₆ alkynyl group, a substituted or unsubstituted C₁-C₆ heteroalkyl group, a substituted or unsubstituted aryl group, -C(O)-(CH₂)ₘ-R^{k}, -C(O)-(CH₂)ₘ-NR^{m}Rⁿ, a group -A-L, and a group L, R^{k} is selected from the group consisting of a substituted or unsubstituted C₁-C₆ alkyl group, a substituted or unsubstituted C₁-C₆ heteroalkyl group, a substituted or unsubstituted aryl group, and a substituted or unsubstituted carboxy group, R^{m} and Rⁿ each independently are hydrogen or a substituted or unsubstituted C₁-C₆ alkyl group, and m is 0 or an integer from 1 to 10;
R⁵ and R⁶ independently are selected from hydrogen, O-R^{o}, wherein R^{o} is a substituted or unsubstituted C₁-C₆ alkyl group;
A is a linker group; and
L is selected from the group consisting of an amino acid residue and a peptide.

The compounds according to the invention have a high radiolytic and chemical stability compared to the known compounds. Further, they allow a quantitative radiolabeling of both Lutetium and Actinium metal ions. In particular, the compounds according to the invention have shown a high stability to human serum albumin as well as faster radiolabeling at mild conditions with radiochemical purities of >95%. Moreover, the compounds according to the invention allow to insert biomolecules to the bispidine moiety, for example via one or more of the following residues: residues R¹, R², R³, R⁴. The biomolecule may be a group L. It may be provided that the compound according to the invention has no or only one group L.

According to the invention a first embodiment of the invention is provided in which X is selected from the first group of meanings given for X and Z is selected from the first group of meanings given for Z. In a second embodiment X is selected from the second group of meanings given for X and Z is selected from the second group of meanings given for Z. In a third embodiment X is selected from the third group of meanings given for X and Z is selected from the third group of meanings given for Z. In this way, it can be ensured that the compounds of general formula I according to the invention can be used as nona- or decadentate ligands for the complexation of metal ions.

R¹ and R² each independently can be a substituted or unsubstituted C₁-C₆ alkyl group. A substituted C₁-C₆ alkyl group may be for example a C₁-C₆ alkyl group having an OH group. In a preferred embodiment the substituted C₁-C₆ alkyl group may be -CH₂-OH. R¹ and R² each independently may be -C(O)-NR^{b}-(CH₂)ₙ-C(O)-O-R^{a}. Preferably, then R^{b} is hydrogen, n = 3, and R^{a} has the meanings given in context with general formula I.

It may be provided that R¹ and R² independently are -C(O)-O-R^{a}, wherein R^{a} has the meanings given in context with general formula I. In a more preferred embodiment, R¹ and R² each are -C(O)-O-R^{a}, wherein R^{a} is hydrogen or a substituted or unsubstituted C₁-C₆ alkyl group. In a particularly preferred embodiment R¹ and R² each are -C(O)-O-R^{a}, wherein R^{a} is a methyl group. Preferably, R¹ and R² have the same meaning.

Preferably, R¹ and R² independently are selected from the group consisting of -CH₂-OH, -COOH, -C(O)-O-CH₃, and -C(O)-NH-(CH₂)₃-C(O)-O-R^{a}, wherein R^{a} has the meanings given in context with general formula I. Preferably, R¹ and R² both are -C(O)-O-CH₃.

Preferably, R¹ and R² have the same meaning. In the following, compounds of general formula I are shown, in which R¹ and R² have the same meaning. In the compound of general formula 1-1 R¹ and R² each are -CH₂-OH: 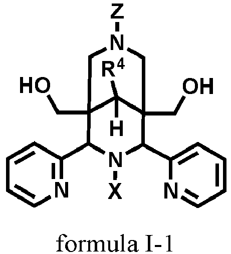 In the compound of general formula 1-2 R¹ and R² each are -C(O)-CH₃: 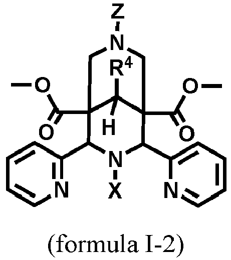 In the compound of general formula 1-3 R¹ and R² each are -C(O)-OH: 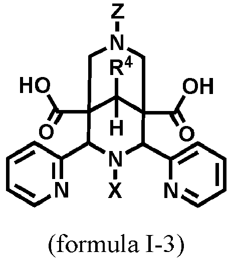 In the compound of general formula 1-4 R¹ and R² each are -C(O)-NH-(CH₂)₃-C(O)-OR^{a}: 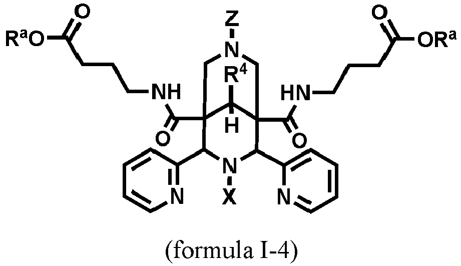 In formulae I-1, I-2, I-3, and 1-4 X and Z each have the meanings given in context with general formula I. In formula 14 R^{a} is selected from the group consisting of hydrogen, a substituted or unsubstituted C₁-C₆ alkyl group, a substituted or unsubstituted aryl group, a group A-L and a group L.

It may be provided that R³ is oxygen or =NR^{d}, wherein R^{d} has the meanings given in context with general formula I. In a preferred embodiment R³ is =N-O-R^{d}, wherein R^{d} has the meanings given in context with general formula I. In a more preferred embodiment R³ is =N-O-R^{d}, wherein R^{d} is a substituted or unsubstituted C₁-C₆ alkyl group. In a particularly preferred embodiment R³ is =N-O-CH₃.

The compound of general formula 1-5 shown below is a preferred compound of general formula I, in which R¹ and R² both are -C(O)-O-CH₃ and R³ is =N-O-CH₃. 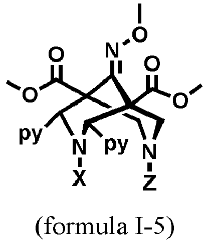 In formula 1-5 X and Z each have the meanings given in context with general formula I. The designation "py" indicates a pyridyl group.

It may be provided that R⁴ is OR^{h}, wherein R^{h} has the meanings given in context with general formula I. Preferably, R⁴ is OR^{h}, wherein R^{h} is selected from the group consisting of hydrogen, a substituted or unsubstituted C₁-C₆ alkyl group, a substituted or unsubstituted C₁-C₆ alkynyl group, -C(O)-(CH₂)ₘ-R^{k}, -C(O)-(CH₂)ₘ-NR^{m}Rⁿ, and a group L, wherein R^{k} is selected from the group, consisting of a substituted or unsubstituted C₁-C₆ alkyl group, a substituted or unsubstituted C₁-C₆ heteroalkyl group, a substituted or unsubstituted aryl group, and -COOH, R^{m} and Rⁿ each independently are hydrogen or a substituted or unsubstituted C₁-C₆ alkyl group, and m is 0 or an integer from 1 to 10. R⁴ can be an SH group. A preferred substituted or unsubstituted aryl group is a substituted or unsubstituted phenyl group. The substituted aryl group may be for example a phenyl group bearing an isothiocyanate group or chlorine.

In a preferred embodiment R⁴ is -OH or -OR^{h}, wherein R^{h} has the meanings given in context with general formula I. In a particularly preferred embodiment R⁴ is -OH or -OR^{h}, wherein R^{h} is a group -A-L or a group L.

In another preferred embodiment R⁴ is selected from the group consisting of -O-C(O)-CH₂-COOH, -O-CH₂-C=CH₃, -O-CH₂-phenyl-isothiocyanate, -O-C(O)-CH₂-NH₂, and -O-C(O)-phenyl-Cl.

The compounds of general formulae I-6, I-7, I-8, I-9, and I-10 shown below are preferred compounds of general formula I, in which R¹ and R² both are -C(O)-O-CH₃. In the compound of general formula 1-6 R⁴ is -O-CH₂-COOH: 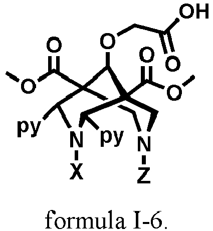 In the compound of general formula 1-7 R⁴ is -O-CH₂-C=CH₃: 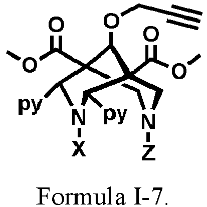 In the compound of general formula 1-8 R⁴ is -O-CH₂-phenyl-isothiocyanate: 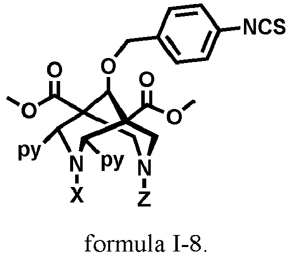 In the compound of general formula 1-9 R⁴ is -O-C(O)-CH₂-NH₂: 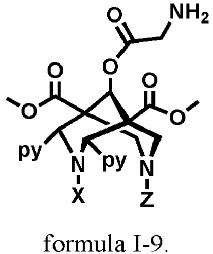 In the compound of general formula 1-10 R⁴ is -O-C(O)-phenyl-Cl: 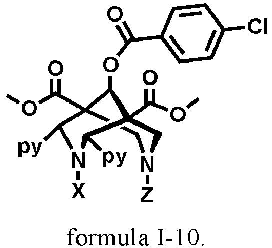 In the formulae I-6, I-7, I-8, I-9, and I-10 X and Z each have the meanings given in context with general formula I. The designation "py" indicates a pyridyl group.

Preferably, R⁵ and R⁶ independently are selected from hydrogen and O-R^{o}, wherein R^{o} is a substituted or unsubstituted C₁-C₆ alkyl group. More preferably, R⁵ and R⁶ independently are selected from hydrogen and O-CH₃. Preferably, R⁵ and R⁶ have the same meaning. Particular preferred, R⁵ and R⁶ are both hydrogen. In another embodiment, R⁵ and R⁶ are both O-CH₃.

In a preferred embodiment,
X and Z have the meanings given in context with general formula I;
R¹ and R² both are -C(O)-O-CH₃;
Y is a group of general formula III;
R⁴ is -OH or -OR^{h}, wherein R^{h} has the meanings given in context with general formula I; and
R⁵ and R⁶ are both hydrogen.

In another preferred embodiment,
X is selected from the first group or the second group;
Z has the meanings given in context with general formula I;
R¹ and R² both are -C(O)-O-CH₃;
Y is a group of general formula III;
R⁴ is -OH or -OR^{h}, wherein R^{h} has the meanings given in context with general formula I; and
R⁵ and R⁶ are both hydrogen.

Group L may be an amino acid residue or a peptide. If the compound according to the invention has no group L, it can be used to chemically bind a biomolecule to a bispidine moiety. For example, this can be done via one or more of the following residues: residues R¹, R², R³, R⁴. The biomolecule may be a group L. An amino acid residue means a group that has a substituted or unsubstituted carboxy group and at least one substituted or unsubstituted amino group. A peptide means a group that has two or more amino acids that are bound to each other via a peptide binding. For example, the peptide may be somatostatin or a somatostatin analogue such as octreotide or octretate. Octretate has the amino acid sequence H-D-Phe-Cys-Phe-D-Trp-Lys-Thr-Cys-Thr-OH. It may be provided that the peptide has no more than 12 amino acid moieties.

In one preferred example, group L is a group of formula L1: 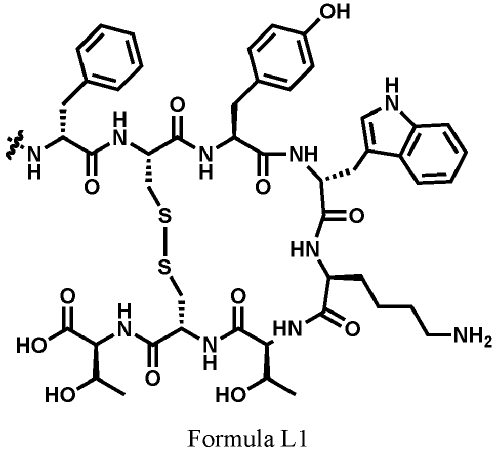 The group of formula L1 is the somatostatin analogue (Tyr³) octreotate (TATE). TATE is a cyclic octapeptide derived from the structure of the octreotide (also known as Sandostatin) and is specific for somatostatin receptors (cell surface receptors) that are overexpressed by some types of cancer. Said peptide is already used in the nuclear-medicine under the name Lutathera and is employed in the diagnosis and therapy of gastroenteropancreatic neuroendocrine tumors.

Group L can be connected by a linker group A to the bispidine unit. One preferred example of a linker group is a group of formula A1: 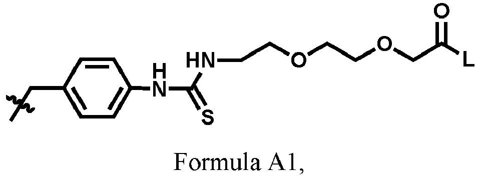 wherein L has the meanings given in context with general formula I. In one embodiment, the group -A-L is a group of formula A1L1:

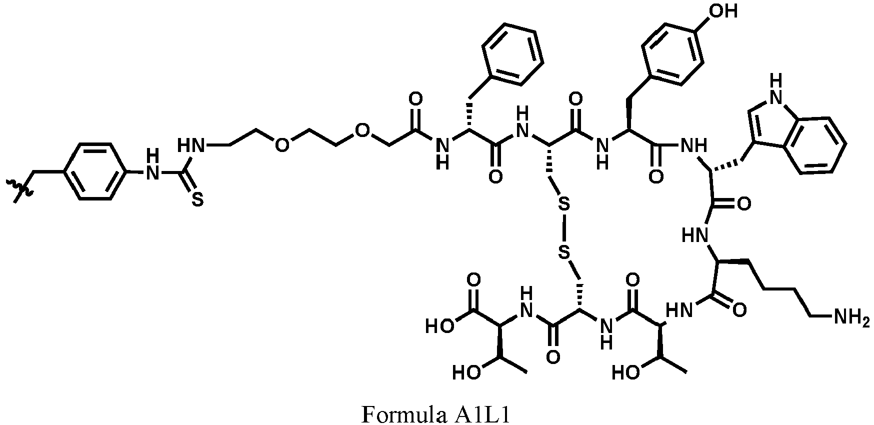

In a first preferred embodiment X is a group of formula 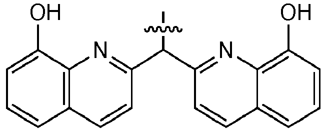 and Z is a group of formula 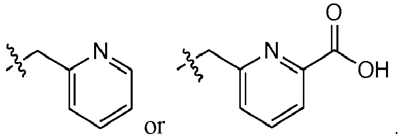 Examples of such compounds are compounds **A1** and **A3** shown in table 1 below.

In a second preferred embodiment X is a group of formula 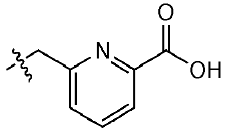 and Z is a group of formula 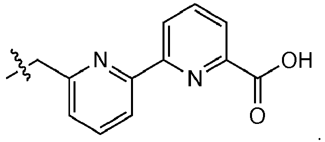 An example of such a compound is compound **A2** shown in table 1 below.

For preparing the compounds of general formula I according to the invention it can be started with a bispidine precursor. The synthesis of a bispidine precursor can follow the way described in Mannich, C. and Mohs, Paul, Berichte der deutschen chemischen Gesellschaft (A and B Series), 63(3), 608-612, 1930. doi: 10.1002/cber.19300630314. The bispidine precursor may be a compound having R¹, R², X and either R³ or R⁴. Subsequently, group Z is introduced. Alternatively, the bispidine precursor may be a compound having R¹, R², Z and either R³ or R⁴. Subsequently, group X is introduced.

Scheme 1 shows a first way of preparing a compound of general formula I: 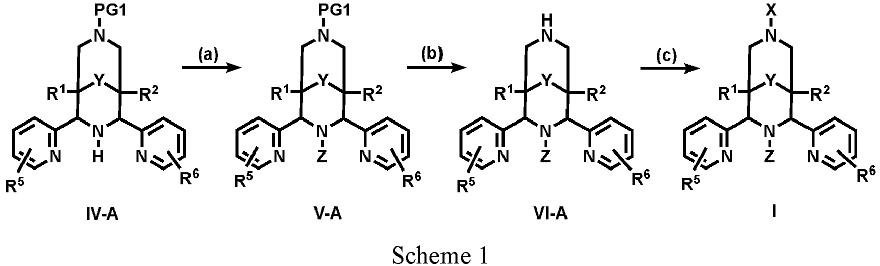 In the formulae IV-A, V-A and VI-A R¹, R², R⁵, R⁶, X, Y, and Z each have the meanings given in context with general formula I. PG1 indicates a first protective group. The first protective group protects the amino functionality. Preferably, the first protective group may be selected from the group consisting of 2,4-dimethoxybenzyl (DMB), *tert-*butyloxycarbonyl (BOC), benzoyl (Bz) and Benzyl. In step (a), group Z is introduced by substituting the hydrogen atom of the bispidine precursor IV-A. In step (b), the first protective group PG1 is cleaved off and replaced by a hydrogen atom. In step (c), group X is introduced by substituting the hydrogen atom. The skilled person is able to carry out the particular steps of this synthesis pathway on the basis of the general knowledge.

Scheme 2 shows a second way of preparing a compound of general formula I: 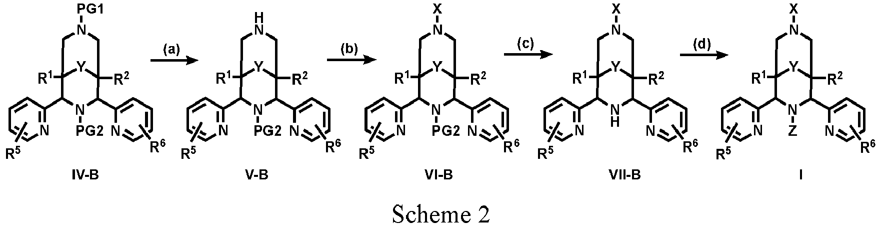 In the formulae IV-B, V-B, VI-B and VII-B R¹, R², R⁵, R⁶, X, Y, and Z each have the meanings given in context with general formula I. PG1 indicates a first protective group. The first protective group protects the amino functionality. Preferably, the first protective group may be selected from the group consisting of 2,4-dimethoxybenzyl (DMB), *tert*-butyloxycarbonyl (BOC), benzoyl (Bz) and Benzyl. PG2 indicates a second protective group. The second protective group protects the amino functionality. Preferably, the first protective group may be selected from the group consisting of 2,4-dimethoxybenzyl (DMB), *tert*-butyloxycarbonyl (BOC), benzoyl (Bz) and Benzyl. The first protective group and the second protective group should be different groups that are cleaved off in different ways. Preferably, the second protective group is different from the first protective group. In step (a), the first protective group PG1 is cleaved off and replaced by a hydrogen atom of the bispidine precursor II-B. In step (b), group X is introduced by substituting the hydrogen atom. In step (c), the second protective group PG2 is cleaved off and replaced by a hydrogen atom. In step (d), group Z is introduced by substituting the hydrogen atom. The skilled person is able to carry out the particular steps of this synthesis pathway on the basis of the general knowledge.

General descriptions of processing modi for preparing the compounds according to the invention are given in section "General Synthesis Methods for the Compounds of general Formula I".

According to the invention further provided is the use of a compound of general formula I according to the invention for the complexation of a metal ion. The metal ion forms the central particle of the complex, the compound of general formula I the ligand of the complex. The metal ion may be a trivalent metal ion. The metal ion may be a radioactive metal ion. Preferably, the metal ion is selected from the group consisting of a lanthanide ion, an actinide ion, an indium ion, a thorium ion, and a bismuth ion. More preferred, the metal ion is selected from the group consisting of a lanthanide(III) ion, an actinide(III) ion (for example a thorium(III) ion), an indium(III) ion, and a bismuth(III) ion. Still more preferred, the metal ion is a lutetium ion or an actinium ion. In particular, the compound according to the invention can be used for the complexation of [¹⁷⁷Lu]Lu ions or [²²⁵Ac]Ac ions. The compound according to the invention binds a radioactive lanthanide ion or a radioactive actinide ion with high stability. The complexes formed are stable in-vitro.

The compounds of general formula I according to the invention in the complexation of the metal ions may form nona- and decadentate ligands around the central particle. The compounds of general formula I according to the invention are nona- or decadentate bispidine derivatives.

According to the invention additionally provided is a method for the complexation of metal ions, in particular of lutetium ions or actinium ions, wherein a compound of general formula I according to the invention is contacted with the metal ion at a reaction temperature in the range of from 20 to 50°C. Preferably, the compound of general formula I according to the invention is contacted with the metal ion at room temperature.

The method according to the invention can be carried out at the reaction temperature of 37°C. Preferably, the method according to the invention is carried out with a protic solvent, for example water. The pH value may be chosen in dependence on the used metal ion. Generally, the reaction may be carried out at a pH value of 5.5 to 7.5, preferably of 6 to 7 and particularly preferred at a pH value of 6 or 7. In case of a bismuth(III) ion, the reaction may be carried out at a pH value of 4.5 to 5. In the case of a [¹⁷⁷Lu]Lu ion or a [²²⁵Ac]Ac ion, the reaction is preferably carried out at a pH value of 5.5 to 7.5, preferably of 6 to 7 and particularly preferred at a pH value of 6 or 7.To adjust the pH value a buffer can be added to the solvent. For example, the buffer may be an ammonium acetate. For example, a solution can be used that contains 0.1 to 0.2, preferably 0.15 M, of buffer.

The method according to the invention can be carried out at ambient pressure. A protective gas is not required.

It may be provided that the molar ratio of the compound of general formula I to a non-radioactive metal ion in the solution is 1 : 1.

A measure for the ratio of a radionuclide to a ligand is the so-called molar specific activity (activity per amount of substance), i. e. the activity of [¹⁷⁷Lu]Lu ions or [²²⁵Ac]Ac ions to the amount of the compound of general formula I. The lower the amount of the compound of general formula I (preferably between 10⁻⁶ and 10⁻⁸ M), the higher the specific activity and the less the compound of general formula I is present in excess. A low specific activity is indicative of the presence of a large amount of "non-radioactive labelled" compound, that might cause side reactions in vivo. The molar specific activity is preferably in a range from 50 to 500 MBq/nmol.

Preferably, the metal ion is provided as an inorganic salt. Inorganic salts of radioactive metal ions may be provided as solution in 0.01 M HCl. For example, the lutetium ions can be provided as LuCl₃, in case of radioactive lutetium ions as [¹⁷⁷Lu]LuCl₃. The actinium ions can be provided as AcNO₃, for example as [²²⁵Ac]AcNO₃.

According to the invention further provided is a complex of a compound of general formula I according to the invention as ligands and a metal ion. The metal ion is preferably selected from the group consisting of a lanthanide ion, an actinide ion, or a bismuth ion. More preferably, the metal ion is a lutetium ion or an actinium ion, particularly preferably, a trivalent lutetium ion or a trivalent actinium ion. The metal ion, for example the lutetium ion or the actinium ion, may be a radioactive metal ion. In particular, the compound according to the invention can be used for the complexation of trivalent metal ions, preferably for the complexation of [¹⁷⁷Lu]Lu ions or [²²⁵Ac]Ac ions.

The complex according to the invention consists of a central particle and one ligand. The metal ion forms the central particle of the complex. The compounds of general formula I according to the invention form a nona- or decadentate ligand. The complex may be a cation. The counter ion to the cation may be selected from the group consisting of Cl⁻, Br, I⁻, NO₃⁻, CF₃SO₂⁻ (OTf), CH₃COO⁻ (OAc), and CF₃CO₂⁻.

Preferred complexes according to the invention are complexes having a [¹⁷⁷Lu]Lu ion as the central particle and a compound A1, A2, A3 or A4 as the ligand. Other preferred complexes according to the invention are complexes having an [²²⁵Ac]Ac ion as the central particle and a compound A1, A2, A3 or A4 as the ligand.

The compounds of general formula I according to the invention advantageously allow to utilize the preferred coordination geometry of the lanthanide ions and actinide ions. They allow a fast complexation due to their open-chain structures. The complexes according to the invention possess a high kinetic stability due to the rigid backbone that is formed by the compounds according to the invention.

The compounds of general formula I according to the invention allow a fast quantitative radiolabeling under mild conditions. The complexes according to the invention possess a high specific molar activity and an excellent serum stability. Radiolabeling means the formation of a complex according to the invention having a radioactive metal ion as the central particle.

It is a particular advantage that radiolabeling can be carried out at 37°C. The complex according to the invention possesses a high kinetic stability in the presence of human serum, so that no trans metalation takes place.

The compounds of general formula I according to the invention allow an easy functionalization of the bispidine moiety with a biomolecule, for example a group L.

The invention allows the preparation of radiopharmaceuticals by fast and efficient radiolabeling with trivalent metal ions, mainly lanthanides and actinides. The complexes prepared retain the required kinetic stability. Thus, the invention allows to provide lanthanide and actinide radiopharmaceuticals. Said radiopharmaceuticals can be used for the diagnosis and therapy of cancer diseases, for example as alpha emitters, beta minus emitters or gamma emitters.

Thus, the complexes according to the invention can be used as a medicament, in particular as a medicament for oncologic diseases. Also, a pharmaceutically acceptable salt of a complex according to the invention can be used as a medicament, in particular as a medicament for oncologic diseases. In one embodiment, the complexes according to the invention can be used as a medicament for the diagnosis and therapy of a carcinoma. Also, a pharmaceutically acceptable salt of a complex according to the invention can be used as a medicament, in particular as a medicament for the diagnosis and therapy of a carcinoma. One example of an oncologic disease is a gastroenteropancreatic neuroendocrine tumor.

Thus, according to the invention it is provided the use of a complex according to the invention, preferably a complex having a [¹⁷⁷Lu]Lu ion or an [²²⁵Ac]Ac ion as the central particle, as a medicament. Thus, a complex according to the invention that has a [¹⁷⁷Lu]Lu ion or an [²²⁵Ac]Ac ion as the central particle can be used as a radiopharmaceutical. Preferably, the medicament is a radiopharmaceutical for use in the nuclear-medical therapy and diagnostics. A complex according to the invention may be used in the nuclear-medical imaging by means of single-photon emission computed tomography (SPECT) (for example in case of a complex having the metal ion Lu) or positron emission tomography (PET). For example, a complex having the metal ion Lu or Tb can be used for this purpose.

In the following, the invention is explained in more detail with the help of examples not intended to limit the invention.

Examples of compounds according to the invention are given in table 1.

**Table 1**

| Compound | Structure | Name |
|---|---|---|
| **A1** | 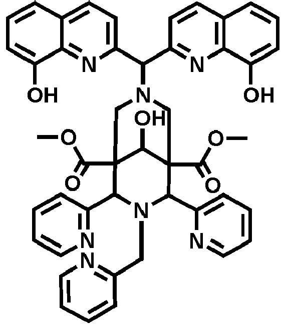 | dimethyl 7-(bis(8-hydroxyquinoline-2-yl)methyl)-9-hydroxy-2,4-di(pyridine-2-yl)-3-(pyridine-2-ylmethyl)-3,7-diazabicyclo[3.3.1]nonane-1,5-dicarboxylate |
| **A2** | 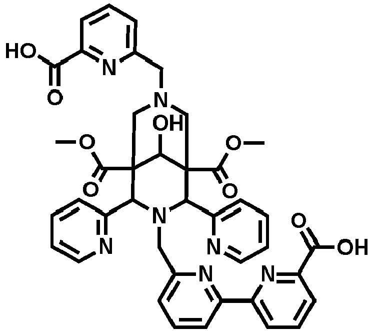 | 6'-((7-((6-carboxypyridine-2-yl)methyl)-9-hydroxy-1,5-bis(methoxycarbonyl)-2,4-di(pyridine-2-yl)-3,7-diazabicyclo[3.3.1]nonane-3-yl)methyl)-[2,2'-bipyridine]-6-carboxylic acid |
| **A3** | 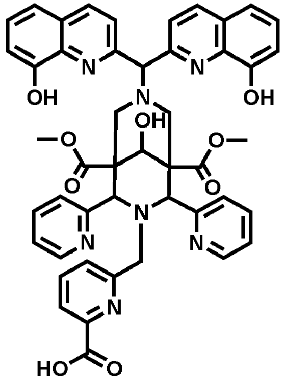 | 6-((7-(bis(8-hydroxyquinoline-2-yl)methyl)-9-hydroxy-1,5-bis(methoxycarbonyl)-2,4-di(pyridine-2-yl)-3,7-diazabicyclo[3.3.1]nonane-3-yl)methyl)picolinic acid |

Compounds **A1**, **A2** and **A3** are exemplary compounds of general formula I.
Compound **A4** shown below is a further example of a compound of general formula I. The designation "py" indicates a pyridyl group. 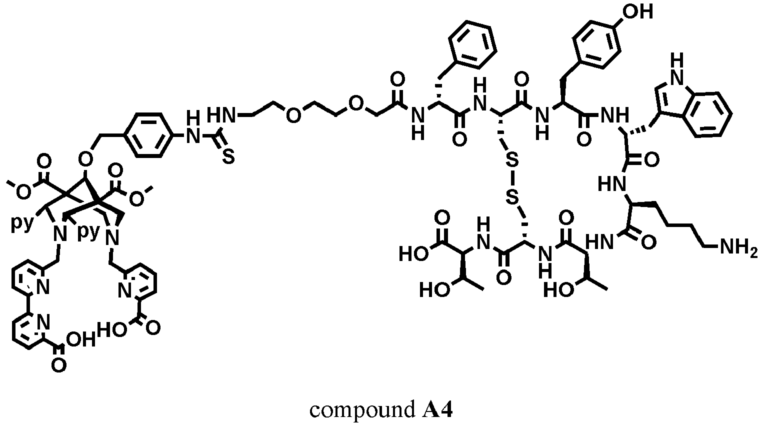 Compound **A4** illustrates the bio-conjugation of a bispidine derivative with an amino acid derivative. **A4** is a derivative of compound **A2** in which the OH group representing residue R⁴ is substituted by a group -O-A-L, wherein -A-L is a group of formula A1L1:

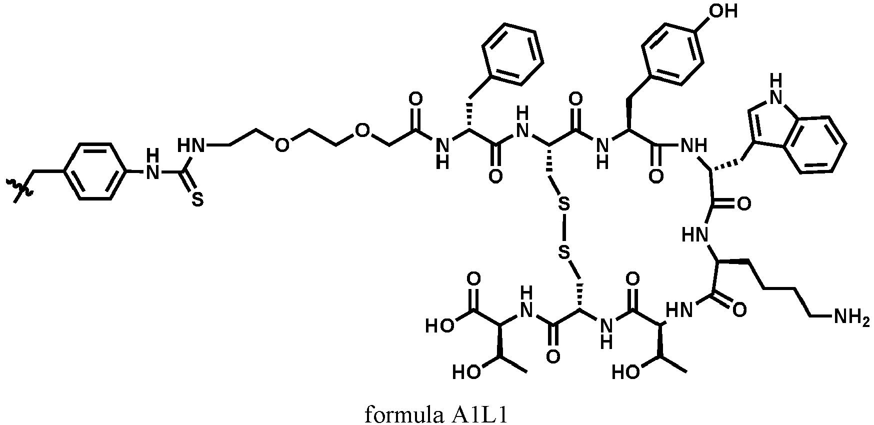

Examples of complexes according to the invention are given in table 2.

**Table 2**

| Complex | Structure | (1) Central Particle (2) Ligand (3) Anion X⁻ |
|---|---|---|
| **K1** = [¹⁷⁷Lu]Lu-**A2** | 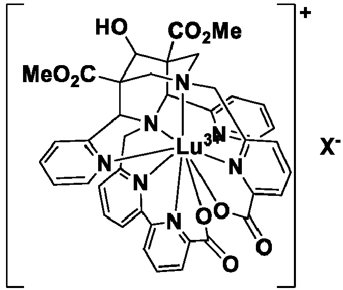 | (1) [¹⁷⁷Lu]Lu³⁺ |
| | | (2) **A2** |
| | | (3) Cl⁻, Br⁻, I⁻, NO₃⁻, CF₃SO₂⁻, CH₃COO⁻ or CF₃CO₂⁻ |
| **K2** = [¹⁷⁷Lu]Lu-**A1** | 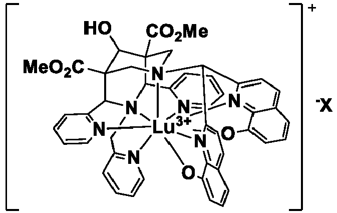 | (1) [¹⁷⁷Lu]Lu³⁺ |
| | | (2) **A1** |
| | | (3) Cl⁻, Br⁻, I⁻, NO₃⁻, CF₃SO₂⁻, CH₃COO⁻ or CF₃CO₂⁻ |
| **K3** = [¹⁷⁷Lu]Lu-**A3** | 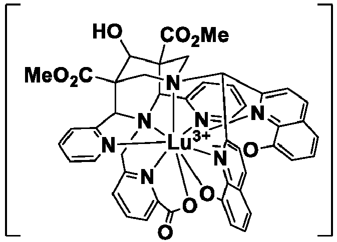 | (1) [¹⁷⁷Lu]Lu³⁺ |
| | | (2) **A3** |
| | | (3) Complex is no cation. |
| **K4** = [²²⁵Ac]Ac-**A1** | 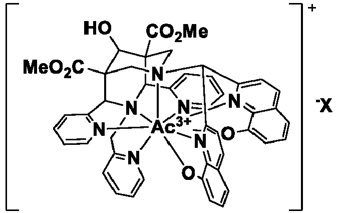 | (1) [²²⁵Ac]Ac |
| | | (2) **A1** |
| | | (3) Cl⁻, Br⁻, I⁻, NO₃⁻, CF₃SO₂⁻, CH₃COO⁻ or CF₃CO₂⁻ |
| **K5** = [²²⁵Ac]Ac-**A2** | 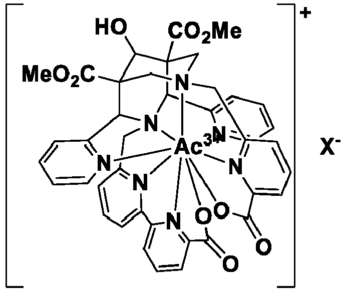 | (1) [²²⁵Ac]Ac |
| | | (2) **A2** |
| | | (3) Cl⁻, Br⁻, I⁻, NO₃⁻, CF₃SO₂⁻, CH₃COO⁻ or CF₃CO₂⁻ |
| **K6** = [²²⁵Ac]Ac-**A3** | 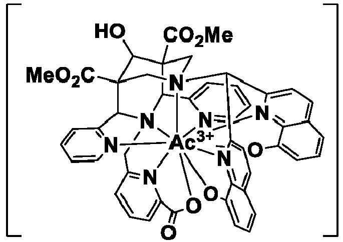 | (1) [²²⁵Ac]Ac |
| | | (2) **A3** |
| | | (3) Complex is no cation |

In table 2 the designation "Me" indicates a methyl group.

The term "alkyl", unless specified otherwise, particularly relates to a monovalent, saturated, aliphatic hydrocarbon group having a branched or unbranched carbon chain with 1 to 12 carbon atoms, preferably 1 to 8 carbon atoms, and particularly preferably 1 to 6 carbon atoms. Examples of alkyl groups include, but are not limited to methyl, ethyl, propyl, isopropyl, isobutyl, *sec*-butyl, *tert*-butyl, pentyl, *n*-hexyl, octyl, dodecyl and the like. The alkyl group may optionally be substituted with one or more substituents, wherein each substituent independently is hydroxy, alkyl, alkoxy, halogen, haloalkyl, amino, mono-alkylamino or dialkylamino, unless specified otherwise.

The term "heteroalkyl" particularly relates to an alkyl residue, as defined herein, wherein one, two or three hydrogen atoms were substituted by a substituent independently selected from the group consisting of -OR^{A}, -NR^{B}R^{C}, and -S(O)ₙR^{D} (in which n is an integer from 0 to 2), with the proviso that the point of attachment of the heteroalkyl residue is a carbon atom, wherein R^{A} is hydrogen, acyl, alkyl, cycloalkyl, or cycloalkylalkyl; R^{B} and R^{C} independently are hydrogen, acyl, alkyl, cycloalkyl, or cycloalkylalkyl; and if n is 0, R^{D} is hydrogen, alkyl, cycloalkyl, or cycloalkylalkyl, and if n is 1 or 2, R^{d} is alkyl, cycloalkyl, cycloalkylalkyl, amino, acylamino, monoalkylamino or dialkylamino. Representative examples include, but are not limited to 2-hydroxyethyl, 3-hydroxypropyl, 2-hydroxy-1-hydroxymethylethyl, 2,3-dihydroxypropyl, 1-hydroxymethylethyl, 3-hydroxybutyl, 2,3-dihydroxybutyl, 2-hydroxy-1-methylpropyl, 2-aminoethyl, 3-aminopropyl, 2-methylsulfonylethyl, aminosulfonylmethyl, aminosulfonylethyl, aminosulfonylpropyl, methylamino-sulfonylmethyl, methylaminosulfonylethyl, methylaminosulfonylpropyl and the like.

The term "cycloalkyl" particularly relates to monovalent, saturated, carbocyclic groups consisting of mono or bicyclic rings. The cycloalkyl group may optionally be substituted with one or more substituents, wherein each substituent independently is hydroxy, alkyl, alkoxy, halogen, haloalkyl, amino, monoalkylamino or dialkylamino, unless specified otherwise. Examples of cycloalkyl components include, but are not limited to cyclopropyl, cyclobutyl, cyclopentyl, cyclohexyl, cycloheptyl, and the like, including partially unsaturated derivatives thereof, such as cyclohexenyl, cyclopentenyl, and the like.

The term "alkenyl", unless specified otherwise, particularly relates to an unsaturated, aliphatic hydrocarbon group having a branched or unbranched carbon chain with 2 to 12 carbon atoms, preferably 2 to 8 carbon atoms, and particularly preferably 2 to 6 carbon atoms, that has at least one olefinic double bond, and more preferably one single double bond. Examples of alkenyl groups include, but are not limited to vinyl, allyl, methallyl, 1,1-dimethylallyl, propenyl, butenyl, pentadienyl, hexenyl, octenyl, and the like. An allyl group is preferred. The alkenyl group may optionally be substituted with one or more substituents, wherein each substituent independently is hydroxy, alkyl, alkoxy, halogen, haloalkyl, amino, monoalkylamino or dialkylamino, unless specified otherwise.

The term "alkynyl", unless specified otherwise, particularly relates to an unsaturated, aliphatic hydrocarbon group having a branched or unbranched carbon chain with 2 to 12 carbon atoms, preferably 2 to 8 carbon atoms, and particularly preferably 2 to 6 carbon atoms, that has at least one olefinic triple bond and more preferably one single triple bond. Examples of alkynyl groups include, but are not limited to acetylenyl, propargyl, *n*-but-2-yn-1-yl, and the like. A propargyl group is preferred. The alkynyl group may optionally be substituted with one or more substituents, wherein each substituent independently is hydroxy, alkyl, alkoxy, halogen, haloalkyl, amino, monoalkylamino or dialkylamino, unless specified otherwise.

The term "alkoxy", unless specified otherwise, particularly relates to a group of formula -OR, in which R is an alkyl group, as defined herein. Examples of alkoxy components include, but are not limited to methoxy, ethoxy, isopropoxy, and the like. The alkoxy group may optionally be substituted with one or more substituents, wherein each substituent independently is hydroxy, alkyl, alkoxy, halogen, haloalkyl, amino, monoalkylamino or dialkylamino, unless specified otherwise.

The term "aryl", unless specified otherwise, particularly relates to a cyclic, aromatic hydrocarbon group consisting of a mono, bi or tricyclic aromatic ring system with 5 to 18 ring atoms, preferably 5 or 6 ring atoms. The aryl group may optionally be a substituted aryl group. Examples of aryl groups include, but are not limited to phenyl, naphthyl, anthracyl, naphthalenyl, phenanthryl, fluorenyl, indenyl, pentalenyl, azulenyl, oxydiphenyl, biphenyl, methylenediphenyl, aminodiphenyl, diphenylsulfidyl, diphenylsulfonyl, diphenylisopropylidenyl, benzodioxanyl, benzofuranyl, benzodioxylyl, benzopyranyl, benzoxazinyl, benzoxazinonyl, benzopiperadinyl, benzopiperazinyl, benzopyrrolidinyl, benzomorpholinyl, methylenedioxyphenyl, ethylenedioxyphenyl, and the like, including partially hydrogenated derivatives thereof. The term "substituted aryl group" particularly relates to an aryl group that is optionally independently substituted with one to four substituents, preferably one or two substituents selected from alkyl, cycloalkyl, heteroalkyl, hydroxyalkyl, halogen, nitro, cyano, isothiocyanato, hydroxy, alkoxy, amino, acylamino, monoalkylamino, dialkylamino, haloalkyl, haloalkoxy, urea, amido, alkanesulfonyl, -COR (in which R is hydrogen, alkyl, phenyl or phenylalkyl), -(CR'R")ₙ-COOR (in which n is an integer from 0 to 5, R' and R" independently are hydrogen or alkyl, and R is hydrogen, alkyl, cycloalkyl, cycloalkylalkyl, phenyl or phenylalkyl) or -(CR'R")ₙ-CONR^{A'}R^{B'} (in which n is an integer from 0 to 5, R' and R" independently are hydrogen or alkyl and R^{A'} and R^{B'} independently are hydrogen, alkyl, cycloalkyl, cycloalkylalkyl, phenyl or phenylalkyl). The aryl group, unless specified otherwise, may be mono or polyvalent, for example mono or divalent.

The term "acyl", unless specified otherwise, relates to a group of formula -C(=O)R, wherein R is hydrogen or alkyl, as defined herein.

The term "halogen" relates to fluorine, chlorine, bromine or iodine.

### General Synthesis Methods for the Compounds of general Formula I

### a) General procedure 1 (GP1): Alkylation of the free amine of a bispidine precursor

A bispidine precursor (1.0 eq.) was suspended in acetonitrile (MeCN). After addition of base (1.0 - 6.0 eq.) and the respective alkylation agent (1.0 - 1.1 eq.) over a given time, the reaction mixture was stirred under reflux (30 min - 16 h). Leftover base was filtered and the solvent of the filtrate was removed under reduced pressure. The leftover solid was dissolved in dichloromethane (DCM) and water and the organic phase collected, followed by extraction of the aqueous phase with DCM. The combined organic phases were dried over Na₂SO₄ and the solvent removed *in-vacuo.* The compounds, comprising the protected bispidine derivative, were purified as described in each procedure.

### General procedure 2 (GP2): Deprotection of DMB- or tBu-protective group

The protected bispidine derviative was dissolved in DCM and equivalent amounts of trifluoroacetic acid was added to the solution. The reaction mixture was stirred under reflux for 16 h, the solvent was removed *in-vacuo* using a separate cooling trap and the crude product was purified as described in each procedure. DMB indicates 2,4-dimethoxybenzyl, *t*Bu a *tert*-butyl group.

### General procedure 3 (GP3): deprotection of benzylic protective group

The protected bispidine derivative was placed in a three necked flask, suspended in EtOAc and 10 wt% of Pd/C was added. The flask was equipped with two adapters with stopcock and a reflux condenser attached to a balloon. The apparatus was flushed with nitrogen and put under vacuum using a water pump. This step was conducted three times, before the same was done using H₂, leaving the apparatus under hydrogen the third time. The reaction mixture was stirred under reflux for at least 16 h until reaction control showed full conversion of the reactant. After filtration over Celite^{®} the solvent was removed under reduced pressure. The product was obtained by ether diffusion into ethanolic solution.

### Example 1

### Synthesis of Compound A1

The method for preparing compound A1 is shown in scheme B1-1. The synthesis of the bispidine fragment 1 follows the process mode described in AF. Bruchertseifer, P. Comba, B. Martin, A. Morgenstern, J. Notni, M. Starke, H. Wadepohl, ChemMedChem 2020**;** BP. Comba, M. Starke, H. Wadepohl, ChemPlusChem 2018, 83, 597-604.

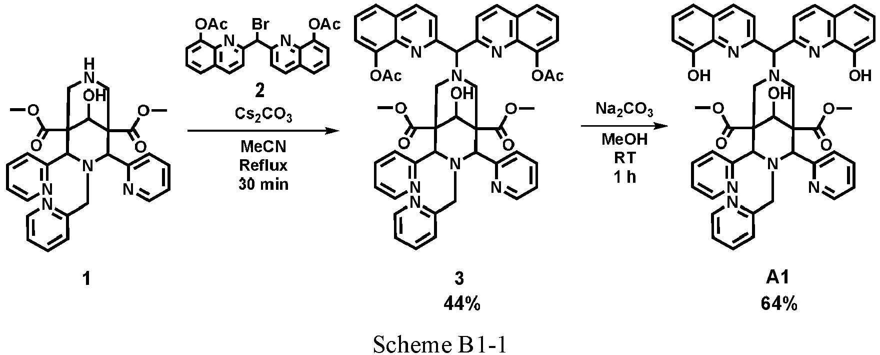

The method for preparing compound 2 needed for preparing compound A1 is shown in scheme B1-2. The alkylation agent 2 was synthesized from literature known compound 5. Compound 5 is described in Y. Yamamoto, A. Miura, A. Kawamata, M. Miura, S. Takei, Bull. Chem. Soc. Jpn. 1978, 51, 3489-3495.

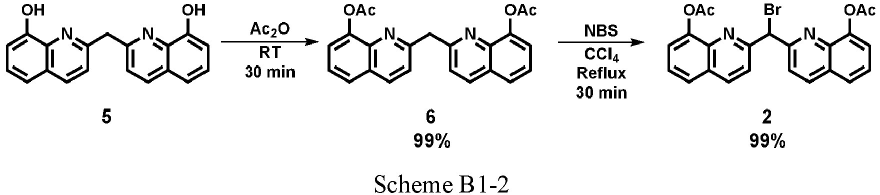

### a) Synthesis of Compound 6

2.00 g of 5 (6.62 mmol, 1.0 eq.) was placed in a flame dried Schlenck tube under N₂, 6.3 ml of acetic anhydride (Ac₂O) (6.75 g, 66.2 mmol, 10.0 eq.) was added and the resulting slurry was stirred at room temperature (RT) for 30 min. Leftover acetic anhydride was removed *in-vacuo* with low heating (50°C). The product **6** was obtained as yellow solid and was used in the next reaction without further purification.

### b) Synthesis of Compound 2

The crude product of **6** (2.56 g, 6.62 mmol, 1.0 eq.) was suspended in CCl₄ (65ml) and 1.18 g of N-bromosuccinimide (NBS) (6.62 mmol, 1.0 eq.) was added. The reaction mixture was refluxed for 30 min and after cooling to room temperature the precipitated solids were removed by filtration. The solvent of the filtrate was removed *in-vacuo* and the leftover solids dissolved in dichloromethane (DCM). The organic phase was washed with first water and then brine and then was dried over Na₂SO₄, filtrated and the solvent removed under reduced pressure. Compound **2** was obtained in quantitative yield as yellow solid (3.08 g, 6.62 mmol, 99 %).
¹H-NMR (200 MHz, 300K, CDCl3): δ = 8.18 (d, J = 8.6 Hz, 2H, Hₐᵣ), 7.85 (d, J = 8.6 Hz, 2H, Hₐᵣ), 7.71 (dd, J = 8.1, 1.6 Hz, 2H, Hₐᵣ), 7.53 (dd, J = 8.1, 7.5 Hz, 2H, Hₐᵣ), 7.43 (dd, J = 7.5, 1.6 Hz, 2H, Hₐᵣ), 6.59 (s, 1H, CHBr), 2.36 (s, 6H, OAc).

### c) Synthesis of Compound 3

The general procedure GP1 was conducted using following amounts: 500 mg of **1** (993 µmol, 1.0 eq.), 647 mg of Cs₂CO₃ (1.99 mmol, 2.0 eq.) and 462 mg of **2** (993 µmol, 1.0 eq.) in 20 ml of acetonitrile (MeCN) and 30 min reaction time. Compound **2** was added over time (10 min). The product was obtained as white solid after crystallization from acetone (390 mg, 437 µmol, 44 %).
**2**·0.25 Cs₂CO₃·1.5 H₂O, [C₅₂.25H53Cs_{0.5}N₇O_{11.5}]: ¹H-NMR (600 MHz, 295K, CDCl₃): δ = 8.44 { 8.41 (m, 1H, Hₐᵣ), 8.41 { 8.37 (m, 2H, H_{py}), 8.04 (d, J = 8.6 Hz, 2H, H_{py}), 7.70 (d, J = 8.5 Hz, 3H, Hₐᵣ), 7.64 (dd, J = 8.2, 1.4 Hz, 2H, Hₐᵣ), 7.47 (t, J = 7.8 Hz, 2H, Hₐᵣ), 7.41 (dd, J = 7.5, 1.4 Hz, 2H, H_{py}), 7.35 (td, J = 7.7, 1.9 Hz, 1H, Hₐᵣ), 7.19 { 7.14 (m, 2H, H_{py}), 6.98 { 6.92 (m, 3H, Hₐᵣ), 6.65 (d, J = 7.8 Hz, 1H, Hₐᵣ), 5.32 (s, 1H, CH_{N7}), 4.74 { 4.70 (m, 3H, CH_{py/CHOH}), 3.66 (s, 2H, CH_{2,py}), 3.56 (s, 6H, CO₂Me), 3.30 (d, J = 12.5 Hz, 2H, CH_{2,ax/eq}), 3.08 (m, 2H, CH_{2,ax/eq}), 2.48 (s, 6H, OAc) ppm. ¹³C-NMR (101 MHz, 295K, CDCl₃): δ = 172.78, 169.91, 159.73, 149.12, 148.71, 147.65, 140.79, 136.28, 135.67, 135.54, 128.85, 126.15, 125.77, 124.58, 124.31, 123.28, 122.52, 121.58, 121.50, 78.32, 71.50, 65.99, 53.76, 52.22, 21.17, 15.41 ppm. ESI-HRMS (pos. DCM/MeOH): m/z [C₅₀H₄₆N₇O₉]⁺ ([M+H]⁺): calc.: 888.3352, exp.: 888.3370; m/z [C₅₀H₄₅N₇NaO₉]⁺ ([M+Na]⁺): calc.: 910.3171, exp.: 910.3189; elemental analysis (Nr.: 45315): calc.: C 60.57, H 4.86, N 9.84; exp.: C 60.66, H 4.83, N 9.96.

### c) Synthesis of Compound A1

To a solution of **3** (212 mg, 238 µmol, 1.0 eq) in Methanol (MeOH) (10 ml) was added 75.9 mg Na₂CO₃ (716 µmol, 3.0 eq.) and the mixture was let to stir for 1 h at room temperature (RT). The solvent was removed under reduced pressure and the leftover solids dissolved in same amounts of dichloromethane (DCM) and water (20 ml). The phases were separated, the aqueous phase extracted with DCM (3x20 ml) and the combined organic phases dried over Na₂SO₄. After filtration the solvent was removed under reduced pressure. To remove coordination cations, the crude product was treated with trifluoroacetic acid (TFA). The TFA salt was obtained by crystallization from ethyl acetate (EtOAc). Compound **A1** was neutralized using pH 7 buffer solution. After DCM extraction (3x50 ml) the combined organic phases were dried over Na₂SO₄ and the solvent was removed *in-vacuo* to furnish **A1** as light-red solid (122 mg, 152 µmol, 64 %).
**A1**·2 MeOH, [C₄₈H₄₉N₇O₉]: ¹H-NMR (400 MHz, 295K, CD₃CN): δ = 8.68 - 8.61 (m, 1H, H_{py}), 8.48 (d, J = 8.7 Hz, 2H, H_{py}), 7.94 (d, J = 8.7 Hz, 2H, H_{py}), 7.73 (d, J = 4.8 Hz, 2H, Hₐᵣ), 7.65 - 7.53 (m, 6H, H_{py,ar}), 7.48 (td, J = 7.7, 1.8 Hz, 1H, H_{py}), 7.24 (dd, J = 7.5, 1.4 Hz, 3H, H_{py}), 7.14 - 7.05 (m, 4H, Hₐᵣ), 6.30 (d, J = 7.8 Hz, 1H, H_{py}), 6.26 (s, 1H, CH_{N7}), 5.02 (s, 2H, CH_{py}), 4.75 (s, 1H, CHOH), 4.02 - 3.93 (m, 4H, CH_{2,ax/eq}), 3.55 (s, 2H, CH_{2,py}), 3.49 (s, 6H, CO₂Me). ¹³C-NMR (101 MHz, 295K, CD₃CN): = 169.95, 155.74, 153.74, 150.60, 150.16,139.23, 138.61, 138.48, 138.04, 130.43, 129.54, 126.71, 124.89, 123.59, 123.46, 119.13, 112.51, 77.93, 71.78, 70.47, 66.22, 53.64, 53.40, 49.81, 15.56 ppm. ESI-HRMS (pos. DCM/MeOH): m/z [C₄₆H₄₂N₇O₇]⁺ ([M+H]⁺): calc.: 804.3140, exp.: 804.3155; m/z [C₄₆H₄₁N₇NaO₇]⁺ ([M+Na]⁺): calc.: 826.2960, exp.: 826.2977; m/z [C₄₆H₄₁KN₇O₇]⁺ ([M+K]⁺): calc.: 842.2699, exp.: 842.2624; elemental analysis (Nr.: 45429): calc.: C 66.42, H 5.69, N 11.30, exp.: C 66.26, H 5.70, N 11.03.

### Example 2

### Synthesis of Compound A2

The method for preparing compound **A2** is shown in scheme B2-1. Piperidone 7, tert-butyl 6-(bromomethyl)picolinate **11,** and 6'-methyl-2,2'-bipyridin carboxylic acid **17** were synthesized according to literature procedure. Compound 7 was synthesized as described in P. Barman, A. K. Vardhaman, B. Martin, S. J. Worner, C. V. Sastri, P. Comba, Angew. Chem. Int. Ed. Engl. 2015, 54, 2095-2099. Compound **11** was synthesized as described in P. Comba, U. Jermilova, C. Orvig, B. O. Patrick, C. F. Ramogida, K. Ruck, C. Schneider, M. Starke, Chem. Eur. J. 2017, 23, 15945-15956. Compound **17** was synthesized as described in M. H. Al-Sayah, R. McDonald, N. R. Branda, Eur. J. Org. Chem. 2004, 2004, 173-182.

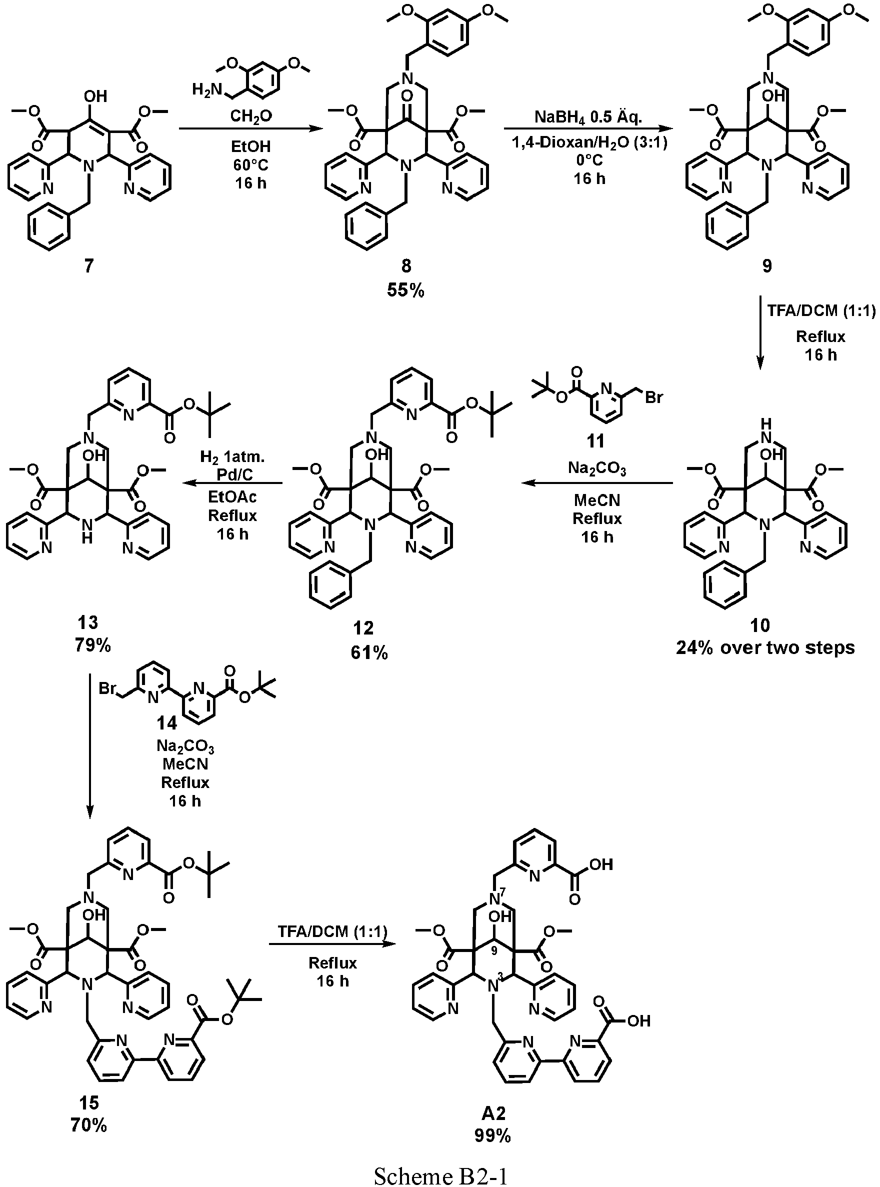

The method for preparing compound 14 needed for preparing compound A1 is shown in scheme B2-2.

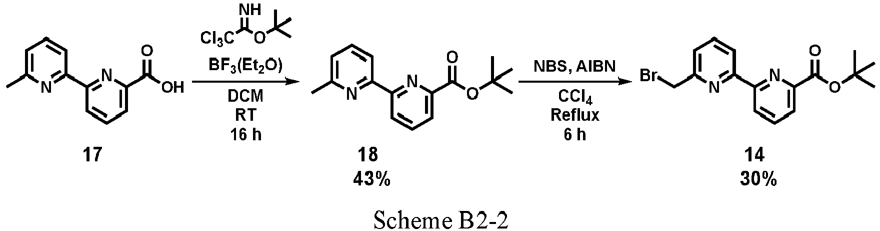

### a) Synthesis of Compound 8

Piperidone 7 (10.2 g, 21.8 mmol, 1.0 equiv.) was suspended in ethanol (EtOH) (130 ml) and heated to 50°C. To this suspension, 2,4-dimethoxybenzylamine (3.80 ml, 4.20 g, 25.1 mmol, 1.2 equiv.) and formaldehyde (3.70 ml, 37% in water, 1.61 g, 50.1 mmol, 2.3 equiv.) were added dropwise and simultaneously by syringe in a period of 5 min. The reaction mixture was refluxed for 16h. After cooling to room temperature, the solvent was removed under reduced pressure, the remaining solid was dissolved in EtOH (15 ml). Layering with diethyl ether yielded **8** as colorless solid (6.87 g, 10.6 mmol, 48%).
¹H-NMR (200 MHz, CDCl₃): δ = 8.49 (ddd, J = 4.9, 1.8, 0.8 Hz, 2H, H_{py}), 8.13 (d, J = 7.9 Hz, 2H, H_{py}), 7.43 (td, J = 7.7, 1.8 Hz, 2H, H_{py}), 7.21 - 7.00 (m, 4H, H_{py,Bn}), 6.84 (dd, J = 7.0, 2.6 Hz, 2H, H_{py}), 6.63 (d, J = 2.4 Hz, 1H, H_{Bn}), 6.44 (dd, J = 8.2, 2.4 Hz, 1H, H_{Bn}), 5.08 (s, 2H, CH_{py}), 4.01 (s, 2H, CH2), 3.87 (s, 2H, CH2), 3.75 (s, 6H, CO2Me), 3.46 (s, 2H, CH2_{Bn/DMB}), 3.39 (s, 2H, CH2_{Bn/DMB}), 3.10 (d, J = 12.9 Hz, 2H, CH2_{ax,eq}), 2.43 (d, J = 12.3 Hz, 2H, CH2_{ax,eq}) ppm.

### b) Synthesis of Compound 9

Compound **8** (6.87 g, 10.6 mmol, 1.0 equiv.) was suspended in 180 ml of a 1,4-dioxane-water mixture (3:1) and cooled to -5°C. A solution of sodium borohydride (201 mg, 5.30 mmol, 0.5 equiv.) in 90 ml of the same solvent mixture was added with a dropping funnel in a period of 20 min. The reaction mixture was allowed to warm up to 0°C and stir at this temperature for 16h. The pH value was set to 1 by adding concentrated sulfuric acid and the solution was stirred for 20 min. The volume was reduced to 50 ml under reduced pressure and an aqueous solution of sodium hydroxide (20 wt%) was added to adjust the pH value to 9. The resulting slurry was stirred for 1h at room temperature before adding DCM (100 ml). The leftover solid was filtered off and the liquid layers separated. Afterwards the aqueous phase was extracted with DCM (3x50 ml), the combined organic layers were dried over anhydrous Na₂SO₄. filtrated and the solvent removed under reduced pressure. Compound **9** was used in the next step without further purification.

**9**·1.5 1,4-dioxane: HRMS (ESI): *m*/*z* calcd for C₃₇H₄₁N₄O₇⁺: 653.2970 [M+H]⁺; found: 653.2971; elemental analysis calcd (%) for C₄₃H₅₂N₄O₁₀: C 65.80, H 6.68, N 7.14; found: C 66.01, H 6.64, N 7.36.

### c) Synthesis of Compound 10

The general procedure 2 (GP2) was conducted using following approach: Crude of **9** (6.82 g) was reacted in 25 ml of DCM with 25 ml of trifluoroacetic acid (TFA). The product was obtained by ether diffusion into methanolic solution resulted in colorless crystals of **10** as TFA salt (3.24 g, 5.25 mmol, 24% over two steps).
**10**·TFA: ¹H-NMR (600 MHz, CDCl₃): δ = 8.67 (s, 2H, H_{py}), 7.76 - 7.55 (m, 2H, H_{py}), 7.25 - 7.22 (m, 2H, H_{py}), 7.20 - 7.15 (m, 1H, H_{Bn}), 7.12 (t, J = 7.3 Hz, 2H, H_{py}), 7.06 (s, 2H, H_{Bn}), 6.61 (d, J = 7.4 Hz, 2H, H_{Bn}), 4.75 (s, 1H, CH_{OH}), 4.53 (d, J = 7.8 Hz, 2H, CH_{py}), 3.70 (d, J = 3.2 Hz, 2H, CH2_{ax,eq}), 3.66-3.56 (m, 2H, CH2_{Bn}), 3.54 (s, 6H, CO2Me) 3.32 (s, 2H, CH2_{ax,eq}) ppm; HRMS (ESI): *m*/*z* calcd for C₂₈H₃₁N₄O₅⁺: 503.2289 [M+H]⁺; found: 503.2286; elemental analysis calcd (%) for C₃₀H₃₁F₃N₄O₇: C 58.44, H 5.07, N 9.09; found: C 58.52, H 5.19, N 9.22.

### d) Synthesis of Compound 12

The general procedure 1 (GP1) was conducted using following approach: 3.24 g of **10** (5.25 mmol, 1.0 equiv.), 3.34 g of Na₂CO₃ (31.5 mmol, 6.0 equiv.) and 1.64 g of *tert*-butyl 6-(bromomethyl)-picolinate **11** (6.04 mmol, 1.2 equiv.) were reacted in 100 ml of MeCN. **12** was recrystallized from hot ethanol (EtOH), yielding the pure compound as colorless solid (2.22 g, 3.20 mmol, 61%).
¹H-NMR (200 MHz, CDCl₃): δ = 8.49 (ddd, J = 4.9, 1.8, 0.9 Hz, 2H, H_{py}), 8.14 (d, J = 7.9 Hz, 2H, H_{py}), 8.07 (dd, J = 7.8, 1.1 Hz, 1H, Hₚₐ), 7.78 (t, J = 7.7 Hz, 1H, Hₚₐ), 7.57 - 7.37 (m, 3H, H_{py,pa}), 7.25 - 7.14 (m, 2H, H_{py}), 7.11 (ddd, J = 7.4, 4.9, 1.2 Hz, 2H, H_{py}), 6.85 (dd, J = 7.4, 1.9 Hz, 2H, H_{py}), 4.53 (s, 2H, CH_{py}), 4.49 (d, J = 5.4 Hz, 1H, CHOH), 3.60 (s, 6H, CO₂Me), 3.58 (s, 2H, CH2_{pa,Bn}), 3.43 (s, 2H, CH2_{pa,Bn}), 2.66 - 2.46 (m, 4H, CH2_{ax,eq}), 1.64 (s, 9H, *t*Bu) ppm.

### e) Synthesis of Compound 13

The general procedure 3 (GP3) was conducted using following approach: Compound **12** (2.22 g, 3.20 mmol, 1.0 equiv.) and Pd/C (222 mg, 10 wt%) were reacted in 140 ml EtOAc. The crude product was dissolved in EtOH (10 ml). Layering with diethyl ether (Et₂O) yielded **13** as colorless solid (1.52 g, 2.53 mmol, 79%).
**13**·0.75MeOH: ¹H-NMR (200 MHz, CDCl₃): δ = 8.36 (d, J = 4.9 Hz, 2H, H_{py}), 8.24 (d, J = 7.1 Hz, 1H, Hₚₐ), 8.12 - 7.85 (m, 2H, H_{py,pa}), 7.71 - 7.52 (m, 2H, H_{py,pa}), 7.28-7.24 (m, 2H, H_{py}), 7.19 (dd, J = 7.6, 4.8 Hz, 2H, H_{py}), 5.03 (s, 2H, CH_{py}), 3.83 (s, 2H, CH2ₚₐ), 3.73 (d, J = 1.0 Hz, 1H, CHOH), 3.66 (s, 6H, CO₂Me), 3.01 (d, J = 5.4 Hz, 4H, CH2_{ax,eq}), 1.61 (s, 9H, *t*Bu) ppm; HRMS (ESI): *m*/*z* calcd for C₃₂H₃₈N₅O₇⁺: 604.2766 [M+H]⁺; found: 604.2727; *m*/*z* calc. for C₃₂H₃₇N₅NaO₇⁺: 626.2585 [M+Na]⁺; found: 626.2587; elemental analysis calcd (%) for C_{32.75}H₄₀N₅O_{7.75}: C 62.67, H 6.42, N 11.16; found: C 62.40, H 6.38, N 11.33.

### f) Synthesis of Compound 15

General procedure 1 (GP1) was followed with following approach: 927 mg of **13** (1.54 mmol, 1.0 equiv), 976 mg of Na₂CO₃ (9.21 mmol, 6.0 equiv.) and 536 mg of *tert*-butyl 6-(bromomethyl)-[2,2'-bipyridine] carboxylate **14** (1.54 mmol, 1.0 equiv.) were reacted in 30 ml of MeCN. The crude product was used in the next step without further purification.
UPLC-MS (APCI): *m*/*z* calcd for C₄₈H₅₄N₇O₉⁺: 872.399 [M+H]⁺; found: 872.530 (peak intensity 100%; retention time: 4.00 min). HRMS (ESI): *m*/*z* calcd for C₄₈H₅₄N₇O₉⁺: 872.3978 [M+H]⁺; found: 872.3968; *m*/*z* calc. for C₄₈H₅₃N₇NaO₉⁺: 894.3797 [M+Na]⁺; found: 894.3787.

### g) Synthesis of Compound A2

The general procedure 2 (GP2) was conducted with following approach: The crude of **15** (1.42 g, 1.0 equiv.) was reacted in 15 ml of DCM and 15 ml of trifluoroacetic acid (TFA). EtOH (10 ml) and 10 ml of diethyl ether were added to the crude solid. The formed precipitate was collected via filtration and the TFA salt of **A2** was obtained as colorless solid (679 mg, 688 µmol, 45% over two steps).
**16**·1.25TFA·2MeOH·0.5MeCN: ¹H-NMR (600 MHz, CD₃OD): δ = 8.70 (s, 1H, Hₐᵣₒₘ), 8.43 (t, J = 7.8 Hz, 2H, Hₐᵣₒₘ), 8.33 - 8.25 (m, 3H, Hₐᵣₒₘ), 8.27 - 8.23 (m, 2H, Hₐᵣₒₘ), 8.07 (s, 1H, Hₐᵣₒₘ), 7.76 - 7.61 (m, 3H, Hₐᵣₒₘ), 7.33 - 7.19 (m, 2H, Hₐᵣₒₘ), 7.07 (s, 2H, Hₐᵣₒₘ), 6.57 (s, 1H, Hₐᵣₒₘ), 5.39 - 5.36 (m, 2H, CH_{py}), 4.85 (s, 1H, CHOH), 4.75 (s, 2H, CH₂bpic), 4.17 (s, 2H, CH2_{ax/eq}), 3.78 (d, J = 12.5 Hz, 2H, CH₂pic), 3.59 (s, 6H, CO₂Me), 3.52 - 3.46 (m, 2H, CH2_{ax/eq}) ppm; ¹³C-NMR (151 MHz, CD₃OD): δ = 208.86, 200.12, 170.61, 168.18, 167.38, 162.85, 157.35, 156.32, 155.53, 151.58, 150.98, 150.91, 149.42, 140.73, 139.76, 138.97, 138.90, 138.71, 126.59, 126.14, 125.28, 121.31, 72.36, 70.79, 62.73, 55.09, 53.98, 53.29, 51.28 ppm;, HRMS (ESI): *m*/*z* calcd for C₄₀H₃₆N₇O₉⁻: 758.2580 [M-H]⁻; found: 758.2580; *m*/*z* calcd for C₄₂H₃₇F₃N₇O₁₁⁻: 872.2509 [M+TFA-H]⁻; found: 872.2506. elemental analysis calcd (%) for C_{45.5}H_{47.75}F_{3.75}N_{7.5}O_{13.5}: C 55.37, H 4.88, N 10.64; found: C 55.28, H 4.73, N 10.65.

### h) Synthesis of tert-butyl 6'-methyl-2,2'bipyridine carboxylate (Compound 18)

6'-Methyl-2,2'-bipyridine carboxylic acid **17** (2.52 g, 11.8 mmol, 1.0 equiv.) was dissolved in 100 ml of DCM. To this solution were added 4.22 ml of tert-butyl 2,2,2-trichloroacetimidate (5.15 g, 23.6 mmol, 2.0 equiv.) and a catalytic amount of Boron trifluoride diethyl etherate (BF₃(Et₂O)) (0.24 ml, 276 mg, 1.97 mmol, 20 µl/mmol acid) and the mixture was let to stir for 16h at room temperature. Sodium carbonate (approx. 150 mg) was added to quench the reaction, the solvent was removed in vacuo and the remaining solid extracted with 200 ml of n-hexane. The extraction process was repeated three times, before removing the solvent in vacuo yielded **18** as colorless solid (1.36 g, 5.00 mmol, 43%).
¹H-NMR (400 MHz, CDCl₃): δ = 8.64 (d, J = 8.8 Hz, 1H, Cₐᵣ), 8.40 (d, J = 7.9 Hz, 1H, Cₐᵣ), 8.03 (dt, J = 7.8, 1.0 Hz, 1H, Cₐᵣ), 7.91 (td, J = 7.8, 1.0 Hz, 1H, Cₐᵣ), 7.73 (dd, J = 8.7, 6.8 Hz, 1H, Cₐᵣ), 7.20 (d, J = 7.6 Hz, 1H, Cₐᵣ), 2.65 (s, 3H, CH₃), 1.66 (s, 9H, *t*Bu) ppm; ¹³C-NMR (101 MHz, CDCl₃): δ = 164.28, 157.87, 154.65, 148.88, 137.80, 137.54, 124.71, 124.00, 118.99, 82.22, 28.28, 27.83 ppm.

### i) Synthesis of tert-butyl 6'-(bromomethyl)-[2,2'-bipyridine]-6-carboxylate (Compound 14)

To a solution of **18** (1.99 g, 7.34 mmol, 1.0 equiv.) in CCl₄ (80 ml) was added N-bromosuccinimide (NBS) (1.31 g, 7.34 mmol, 1.0 equiv.) and azobisisobutyronitrile (AIBN) (199 mg, 1.22 mmol, 10 wt%). The resulting suspension was refluxed for 6h, cooled to room temperature, filtrated and the solvent removed in vacuo. Compound **14** was obtained as colorless solid after purification with column chromatography (SiO₂, gradient from 100% petroleum ether to 15% ethyl acetate) in 30% yield (777 mg, 2.22 mmol).
**14:** ¹H-NMR (200 MHz, CDCl₃): δ = 8.64 (dd, J = 7.8, 1.3 Hz, 1H, Cₐᵣ), 8.52 (dd, J = 7.9, 1.0 Hz, 1H, Cₐᵣ), 8.06 (dd, J = 7.7, 1.3 Hz, 1H, Cₐᵣ), 7.93 (t, J = 7.7 Hz, 1H, Cₐᵣ), 7.84 (t, J = 7.8 Hz, 1H, Cₐᵣ), 7.48 (dd, J = 7.7, 1.0 Hz, 1H, Cₐᵣ), 4.63 (s, 2H, CH₂), 1.67 (s, 9H, *t*Bu) ppm; ¹³C-NMR (151 MHz, CDCl₃): δ = 164.06, 156.19, 148.82, 138.54, 138.13, 138.11, 125.13, 124.32, 124.21, 123.49, 121.24, 82.47, 33.79, 28.28 ppm; HRMS (ESI): *m*/*z* calcd for C₁₆H₁₇BrN₂NaO₂⁺: 371.0366 [M+Na]⁺; found: 371.0363; elemental analysis calcd (%) for C₁₆H₁₇BrN₂O₂: C 55.03, H 4.91, N 8.02; found: C 54.73, H 4.85, N 8.55.

### Example 3

### Synthesis of Compound 21

Compound 15 prepared in example 2, step f can be used for preparing compound **21.** Here, the OH group forming residue R⁴ is subjected to a functionalization. The preparation of compound **21** is shown in scheme B3-1. Compound **21** can be converted into a compound according to the invention by cleavage of the tert-butyl protective group.

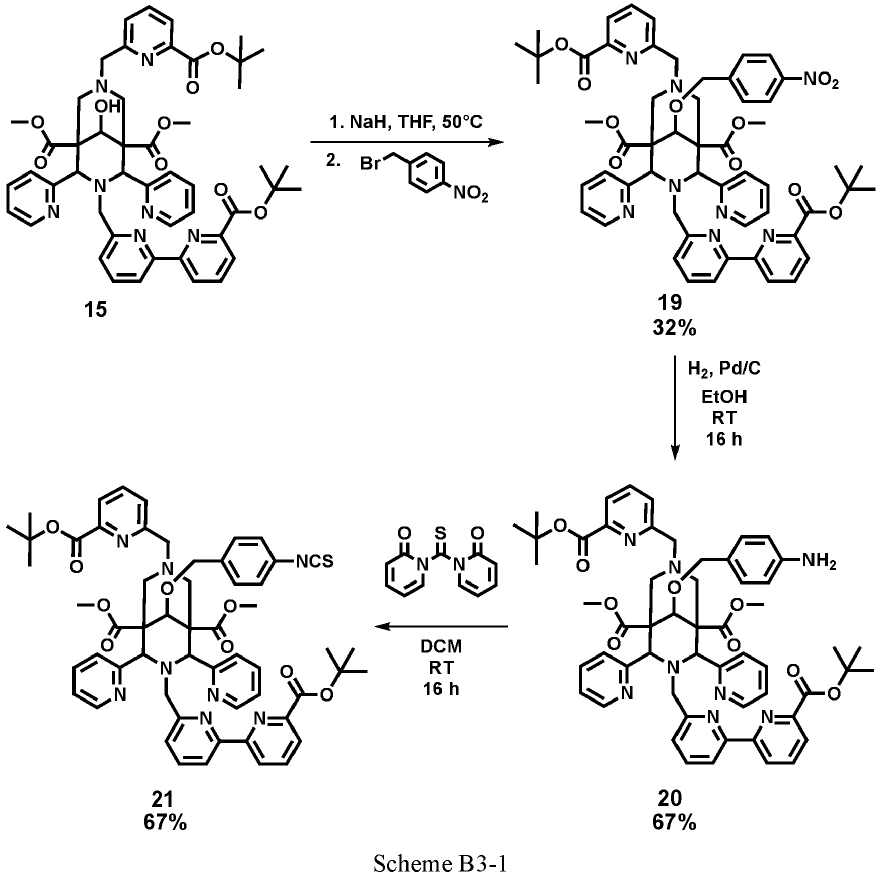

### a) Synthesis of Compound 19

Under nitrogen and in a flame dried Schlenck flask, **15** (200 mg, 229 µmol, 1.0 eq.) was dissolved in dry tetrahydrofuran (THF). After addition of sodium hydride (NaH) (60 wtf%, 7.20 mg, 298 µmol, 1.3 eq.) the reaction mixture was stirred for 1 h at 50°C, until gas evolution stopped. 64.4 mg of 4-nitrobenzylbromide (298 µmol, 1.3 eq.) was added and the mixture was stirred at 50°C for 24 h. The reaction was stopped by adding water (5 ml), followed by extraction with DCM (3x50 ml). The combined organic phases were dried over Na₂SO₄, filtrated and the solvent removed under reduced pressure. The product **19** was obtained by column chromatography (C₁₈-SiO₂, gradient from 80 % water to 100 % MeOH) as orange solid (75.2 mg, 74.7 µmol, 32 %).
¹H-NMR (400 MHz, 295K, CDCl₃): = 9.06 (d, J = 7.8 Hz, 1H, H_{py}), 8.52 - 8.40 (m, 3H, H_{pa,bpa}), 8.17 (d, J = 7.8 Hz, 2H, H_{pa,bpa}), 8.11 (d, J = 8.7 Hz, 2H, H_{Bn-NO2}), 8.05 (td, J = 7.4, 1.1 Hz, 2H, H_{pa,bpa}), 7.98 (t, J = 7.8 Hz, 1H, H_{pa/bpa}), 7.74 (t, J = 7.7 Hz, 1H, H_{py}), 7.68 (t, J = 7.7 Hz, 1H, H_{py}), 7.47 (td, J = 7.6, 1.8 Hz, 3H, H_{py,pa/bpa}), 7.28 (d, J = 8.7 Hz, 2H, H_{Bn-NO2}), 7.09 (ddd, J = 7.5, 4.8, 1.2 Hz, 2H, H_{py}), 6.77 (d, J = 7.5 Hz, 1H, H_{py}), 5.08 (s, 2H, CH_{2,Bn-NO2}), 4.65 (s, 2H, CH_{py}), 4.53 (s, 1H, CHOH), 3.66 (s, 2H, CH_{2,bpa}), 3.57 (s, 2H, CH_{2,pa}), 3.47 (s, 6H, CO₂Me), 2.58 (s, 4H, CH_{2,ax/eq}), 1.68 (s, 9H, *t*Bu), 1.65 (s, 9H, *t*Bu). ¹³C-NMR (101 MHz, 295K, CDCl₃): = 172.64, 164.94, 164.32, 160.21, 159.69, 158.52, 156.77, 154.76, 154.67, 148.58, 147.40, 147.18, 146.73, 146.54, 137.24, 137.22, 137.00, 136.95, 136.41, 135.96, 128.46, 127.48, 126.95, 125.76, 124.41, 123.58, 122.66, 119.77, 82.28, 82.19, 81.71, 77.48, 76.84, 73.77, 70.09, 65.37, 53.57, 52.67, 51.95, 50.31, 28.41, 28.31. ESI-HRMS (pos. DCM/MeOH): m/z [C₅₅H₅₉N₈O₁₁]⁺ ([M+H]⁺): calc.: 1007.4298, exp.: 1007.4361; m/z [C₅₅H₅₈N₈NaO₁₁]⁺ ([M+Na]⁺): calc.: 1029.4117, exp.: 1029.4171.

### b) Synthesis of Compound 20

128 mg of **19** (127 µmol, 1.0 eq.) was placed into a three necked flask and suspended in 40 ml of ethanol (EtOH), Pd/C (26.0 mg, 20wt%) was added and the flask equipped with two valves with stopcock and a reflux condenser attached to a balloon. The apparatus was flushed three times with N₂ and put under vacuum using a water pump. The same procedure was done using H₂, leaving the apparatus under hydrogen the third time. The reaction mixture was let to stir for 48h at room temperature, before it was filtrated through Celite^{®}. The solvent was removed under reduced pressure and the solids extracted with Et₂O, yielding the product **20** as pale-yellow solid (83.6 mg, 85.6 µmol, 67 %).
¹H-NMR (600 MHz, 295K, CDCl₃): δ = 9.08 (d, J = 7.4 Hz, 1H, H_{py}), 8.47 - 8.44 (m, 2H, H_{pa,bpa}), 8.37 (d, J = 8.0 Hz, 1H, H_{pa/bpa}), 8.16 (d, J = 7.6 Hz, 2H, H_{pa,bpa}), 8.07 - 8.01 (m, 3H, H_{pa,bpa}), 7.76 (t, J = 7.8 Hz, 1H, H_{py}), 7.70 (t, J = 7.7 Hz, 1H, H_{py}), 7.47 (td, J = 7.6, 1.7 Hz, 2H, H_{py}), 7.10 (dd, J = 7.2, 5.0 Hz, 2H, H_{py}), 6.89 (d, J = 8.3 Hz, 2H, H_{Bn-NH2}), 6.74 (d, J = 7.9 Hz, 1H, H_{py}), 6.56 (d, J = 8.3 Hz, 2H, H_{Bn-NH2}), 5.12 (d, J = 3.8 Hz, 2H, CH_{2,Bn-NH2}), 4.53 (s, 1H, CHOH), 4.34 (s, 2H, CH_{py}), 3.64 (s, 2H, CH_{2,bpa}), 3.56 (s, 6H, CO₂Me), 3.48 (s, 2H, CH_{2,pa}), 2.53 - 2.45 (m, 4H, CH_{2,ax/eq}), 1.66 (s, 9H, *t*Bu), 1.63 (s, 9H, *t*Bu). ESI-HRMS (pos. DCM/MeOH): m/z [C₅₅H₆₀N₈NaO₉]⁺ ([M+Na]⁺): calc.: 999.4375, exp.: 999.4403.

### Synthesis of Compound 21

20 mg (20.5 µmol, 1 eq.) of **20** and 4.8 mg (20.5 µmol, 1 eq.) of 1,1-thio-carbonyldi-2-(1H)-pyridone were place into an oven dried round-bottom flask and dissolved in dry DCM. The reaction mixture was stirred for 24 h at room temperature. The solvent was removed under reduced pressure and purified by preparative HPLC using a Jupiter Proteo (250 mm x 21.2 mm, 4 µm, 90 Å) with a flow rate of 10 mL/min. The solvents used were H₂O + 0.1% TFA (A) and acetonitrile + 0.1% TFA (B). The gradient was applied as follows: 5 min 50% B, 50% to 90% B in 20 min, 90 to 95% B in 1 min, 5 min 95% B. The retention time is 22.8 min. The fractions were combined and lyophilized to yield product **21** as a colorless powder (14 mg, 13.8 µmol, 67%).
ESI-MS (pos. H₂O/CH₃CN): m/z ([M+H]⁺): calc.: 1019.41, exp.: 1019.06; ([M+Na]⁺): calc.: 1041.4, exp.: 1041.00; ([M+H-*t*Bu]⁺): calc.: 963.07, exp.: 963.07; ([M+2H-2*t*Bu]⁺): calc.: 906.98, exp.: 906.98. IR (diamond, ATR): v (-NCS) = 2095 cm⁻¹.

### Example 4

### Synthesis of Compound A3

The method for preparing compound **A3** is shown in scheme B4-1. Compound **22** was synthesized as described in AR. Haller, H. Unholzer, Arch. Pharm. 1971, 304, 654-659, and BA. Samhammer, U. Holzgrabe, R. Haller, Arch. Pharm. 1989, 322, 551-555.

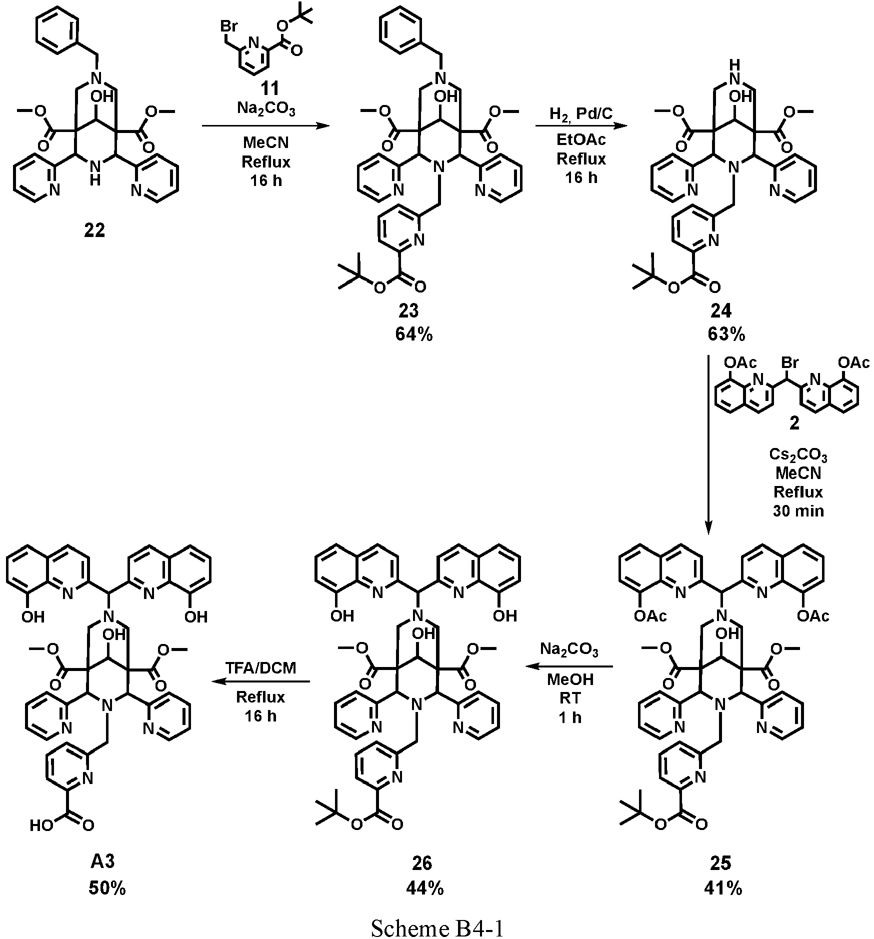

### a) Synthesis of Compound 22

General procedure 1 (GP1) was conducted with the following approach: 1.00 g of **22** (1.99 mmol, 1.0 eq.), 1.27 g Na₂CO₃ (11.9 mmol, 6.0 eq.) and 542 mg **11** (1.99 mmol, 1.0 eq.) were reacted for 16 h in 40 ml acetonitrile (MeCN). The product was obtained by crystallization from acetone as colorless solid (888 mg, 1.28 mmol, 64 %).
[C₃₉H₄₃N₅O_{7]}: ¹H-NMR (600M Hz, 295K, CDCl₃): δ = 8.35 (dd, J = 5.0, 1.8 Hz, 2H, H_{py}), 7.84- 7.81 (m, 2H, H_{py}), 7.68 (d, J = 7.7 Hz, 1H, Hₚₐ), 7.43 - 7.40 (m, 2H, H_{Bn}), 7.39 -7.36 (m, 2H, H_{Bn}), 7.35 - 7.32 (m, 2H, H_{pa,Bn}), 7.29 (td, J = 7.7, 1.9 Hz, 2H, H_{py}), 6.99 (ddd, J = 7.5, 4.8, 1.2 Hz, 2H, H_{py}), 6.67 (d, J = 7.8 Hz, 1H, Hₚₐ), 4.91- 4.88 (m, 1H, CHOH), 4.77 (s, 2H, CH_{py}), 3.64 (d, J = 1.7 Hz, 2H, CH_{2,pa}), 3.62 (s, 6H, CO₂Me), 3.36 (s, 2H, CH_{2,Bn}), 2.64 (s, 2H, CH_{2,ax/eq}), 2.45 (s, 2H, CH_{2,ax/eq}), 1.70 (s, 9H, *t*Bu) ppm. ¹³C-NMR (151 MHz, 295K, CDCl₃): = 172.67, 164.49, 159.13, 157.92, 148.96, 148.92, 148.26, 137.85, 136.20, 135.49, 130.65, 129.94, 128.30, 128.18, 127.29, 126.52, 124.78, 122.55, 122.51, 121.70, 81.74, 72.46, 66.77, 64.01, 53.24, 52.21, 52.02, 51.74, 49.33, 49.10, 28.33 ppm. ESI-HRMS (pos. DCM/MeOH): m/z [C₃₉H₄₃N₅O₇]⁺ ([M+H]⁺): calc.: 694.3244, exp.: 694.3244; elemental analysis (Nr.: 45409): calc.: C 67.52, H 6.25, N 10.09; exp.: C 67.31, H 6.21, N 10.11.

### b) Synthesis of Compound 24

General procedure 3 (GP3) was conducted using following approach: 888 mg of **23** (1.28 mmol) and 88.8 mg of Pd/C were reacted in 60 ml ethyl acetate (EtOAc). The crude product was purified by washing with Et₂O using an ultrasonic bath (10 min) and subsequent filtration. After drying *in-vacuo* the product **24** was obtained as colorless solid (488 mg, 808 µmol, 63 %).
**24**·0.5 H2O, [C₃₂H₃₈N₅O_{7.5}]: ¹H-NMR (600 MHz, 295K, CDCl₃): δ = 8.55 (s, 2H, H_{py}), 7.78 (d, J = 7.2 Hz, 1H, Hₐᵣ), 7.58 (d, J = 47.8 Hz, 4H, Hₐᵣ), 7.15 (d, J = 6.4 Hz, 2H, Hₐᵣ), 6.87 (s, 1H, Hₐᵣ), 4.95 - 4.44 (m, 3H, CH_{py}/CHOH), 3.58 (s, 2H, CH_{2,pa}), 3.47 (s, 6H, CO₂Me), 3.20 - 3.04 (m, 4H, CH_{2,ax/eq}), 1.65 (s, 9H, *t*Bu) ppm. ¹³C-NMR (101 MHz, 295K, CDCl3): δ = 172.93, 164.37, 157.75, 149.47, 148.82, 136.59, 136.22, 123.02, 81.95, 73.60, 70.93, 65.98, 51.94, 51.64, 42.34, 37.44, 28.34 ppm. ESI-HRMS (pos. DCM/MeOH): m/z [C₃₂H₃₈N₅O₇]⁺ ([M+H]⁺): calc.: 604.2766, exp.: 604.2768 elemental analysis (Nr.: 45330): calc.: C 62.73, H 6.25, N 11.43; exp.: C 62.62, H 6.04, N 11.33.

### c) Synthesis of Compound 25

General procedure 1 (GP1) was followed with the following approach: 431 mg of **24** (713 µmol, 1.0 eq.), 232 mg Cs₂CO₃ (713 µmol, 1.0 eq.) and 332 mg of **2** (713 µmol, 1.0 eq.) were reacted for 30 min in 20 ml MeCN. The bromide **2** was added over a time period of 10 min. The crude product was purified by use of column chromatography (C₁₈-SiO₂, gradient from 80 % water to 100 % MeOH) and yielded the **25** as yellow solid (290 mg, 293 µmol, 41 %).
**25**·5 H₂O·0.25 MeCN, [C_{55.5}H_{63.75}N_{7.25}O₁₆]: ¹H-NMR (600 MHz, 295K, CDCl₃): δ = 8.36 (d, J = 8.5 Hz, 2H, H_{py}), 7.99 (s, 2H, Hₐᵣ), 7.93 (d, J = 6.8 Hz, 1H, Hₐᵣ), 7.83 (d, J = 7.7 Hz, 2H, H_{py}), 7.67 (t, J = 7.9 Hz, 1H, Hₐᵣ), 7.62 - 7.44 (m, 7H, Hₐᵣ), 7.22 (s, 2H, H_{py}), 7.04 (s, 2H, H_{py}), 6.25 (s, 1H, Hₐᵣ), 6.13 (s, 1H, CH_{N7}), 5.03 (s, 3H, CH_{py}/CHOH), 4.41 (s, 2H, CH_{2,ax/eq}), 4.07 (s, 2H, CH_{2,ax/eq}), 3.63 (s, 6H, CO₂Me), 3.60 (s, 2H, CH_{2,pa}), 2.38 (s, 6H, OAc), 1.74 (s, 9H, *t*Bu) ppm. ¹³C-NMR (151 MHz, 295K, CD₂Cl₂): δ = 169.97, 169.09, 163.93, 148.92, 148.69, 147.79, 147.65, 141.21, 137.96, 136.95, 129.28, 128.07, 126.00, 123.77, 123.26, 122.92, 117.65, 103.49, 82.67, 66.00, 53.09, 52.73, 49.30, 28.48, 26.81, 21.28 ppm. ESI-HRMS (pos. DCM/MeOH): m/z [C₅₅H₅₄N₇O₁₁]⁺ ([M+H]⁺): calc.: 988.3876, exp.: 988.3880; m/z [C₅₅H₅₃N₇NaO₁₁]⁺ ([M+Na]⁺): calc.: 1010.3695, exp.: 1010.3703; elemental analysis (Nr.: 45337): calc.: C 61.25, H 5.90, N 9.33; exp.: C 61.07, H 5.75, N 9.30.

### d) Synthesis of Compound 26

To a solution of **25** (1.20 g, 1.21 mmol, 1.0 eq) in methanol (MeOH) (50 ml) was added 386 mg Na₂CO₃ (3.64 mmol, 3.0 eq.) and the mixture was let to stir for 1 h at room temperature (RT). The solvent was removed under reduced pressure and the leftover solids dissolved in same amounts of DCM and water (20 ml). The phases were separated, the aqueous phase extracted with DCM (3x20 ml) and the combined organic phases dried over Na₂SO₄. After filtration the solvent was removed under reduced pressure and the crude product of **26** was used in the next reaction without further purification.
**26**·2 H2O, [C₅₁H₅₃N₇O₁₁]: ¹H-NMR (200 MHz, 300K, CDCl₃): δ = 8.30 - 8.19 (m, 3H, H_{ar,py}), 7.96 (d, J = 8.5 Hz, 2H, H_{py}), 7.87 - 7.77 (m, 2H, Hₐᵣ), 7.69 (d, J = 7.0 Hz, 2H, Hₐᵣ), 7.51 -7.34 (m, 5H, Hₐᵣ), 7.32 - 7.20 (m, 2H, Hₐᵣ), 7.12 (dd, J = 7.3, 1.6 Hz, 2H, H_{py}), 6.98 - 6.86 (m, 2H, H_{py}), 6.73 (d, J = 8.0 Hz, 1H, Hₐᵣ), 5.20 (s, 1H, CH_{N7}), 4.73 (s, 1H, CHOH), 4.66 (s, 2H, CH_{py}), 3.59 (s, 2H, CH_{2,pa}), 3.50 (s, 6H, CO₂Me), 3.15 (d, J = 11.9 Hz, 2H, CH_{2,ax/eq}), 2.90 (s, 2H, CH_{2,ax/eq}), 1.66 (s, 9H, *t*Bu) ppm. ESI-HRMS (pos. DCM/MeOH): m/z [C₅₁H₅₀N₇O₉]⁺ ([M+H]⁺): calc.: 904.3670, exp.: 904.3705; m/z [C₅₁H₄₉N₇NaO₉]⁺ ([M+Na]⁺): calc.: 926.3489, exp.: 926.3520; elemental analysis (Nr.: 45355): calc.: C 65.16, H 5.68, N 10.43, exp.: C 65.26, H 5.46, N 10.56.

### e) Synthesis of Compound 27

General procedure 2 (GP2) was conducted using following approach: 240 mg of **26** (265 µmol) was reacted in 10 ml of DCM and 10 ml of trifluoroacetic acid. Product **27** was obtained by precipitation with diethyl ether (Et₂O) from acetonic solution (128mg, 133 µmol, 50 %).
**27**·1.5 H₂O·HOAc, [C₄₉H₄₈N₇O_{12,5}]: 1H-NMR (400 MHz, 295K, CD₃CN): δ = 8.47 (d, J = 8.8 Hz, 2H, H_{py}), 7.97 (d, J = 7.7 Hz, 2H, H_{py}), 7.94 - 7.87 (m, 2H, Hₐᵣ), 7.86 - 7.79 (m, 1H, Hₐᵣ), 7.74 - 7.67 (m, 2H, Hₐᵣ), 7.67 - 7.59 (m, 3H, Hₐᵣ), 7.58 - 7.51 (m, 2H, Hₐᵣ), 7.24 (d, J = 7.5 Hz, 4H, H_{py}), 7.16 (s, 2H, Hₐᵣ), 6.57 (s, 1H, Hₐᵣ), 6.30 (s, 1H, CH_{N7}), 5.13 (s, 2H, CH_{py}), 4.73 (s, 1H, CHOH), 4.05 - 3.92 (m, 2H, CH_{2,ax/eq}), 3.86 - 3.72 (m, 2H, CH_{2,ax/eq}), 3.59 (s, 2H, CH_{2,pa}), 3.50 (s, 6H CO₂Me) ppm. ¹³C-NMR (101 MHz, 295K, CD₃CN): δ = 166.64, 156.07, 153.70, 150.29, 146.56, 139.32, 138.57, 130.43, 124.86, 123.65, 119.12, 112.52, 53.64, 49.89 ppm. ESI-HRMS (pos. DCM/MeOH). m/z [C₄₇H₄₁KN₇O₉]⁺ ([M+K]+): calc.: 886.2597, exp.: 886.2528 m/z [C₄₇H₄₁N₇NaO₉]⁺ ([M+Na]⁺): calc.: 870.2858, exp.: 870.2755; elemental analysis (Nr.: 45315): calc.: C 62.95, H 5.17, N 10.49; exp.: C 63.12, H 5.17, N 10.69.

### Example 5

### Synthesis of Compound A4

The synthesis of compound **A4** illustrates the introduction of an amino acid derivative as residue R⁴. The synthesis of compound A4 is shown in scheme B5-1. The synthesis starts with compound **21** that is shown in scheme B5-1 in a varied form. Here, picture A and picture B both are the reproduction of compound **21.** 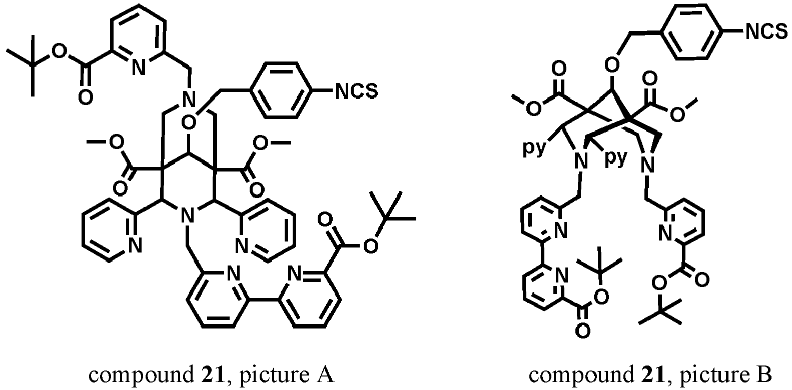 In picture B the designation "py" indicates a pyridyl group. 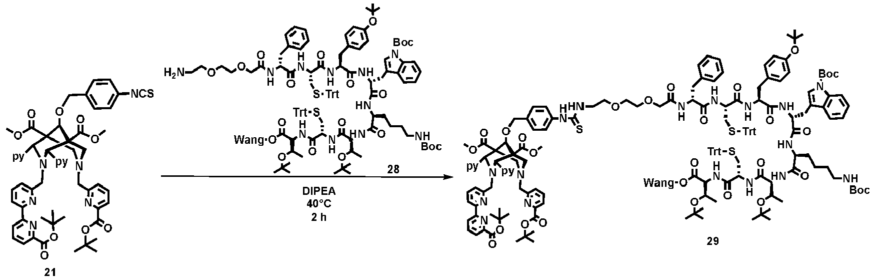 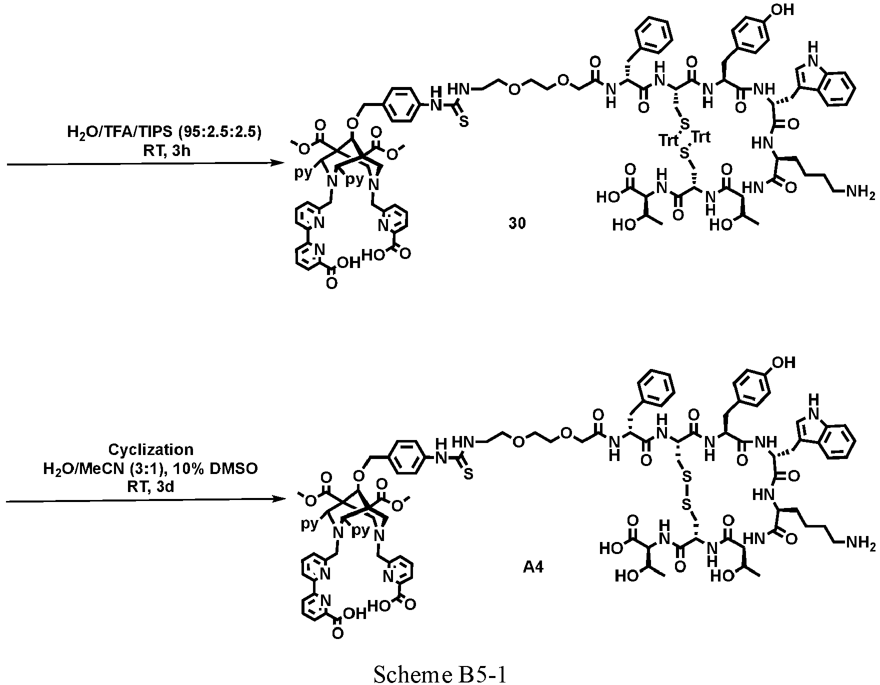 The designation "Wang" used in compounds 28 and 29 indicates a resin for attaching carboxylic acids for further functionalization (S. S. Wang, J. Am. Chem. Soc., 1973, 95, 1328).

Compound **28** and compound **A4** were conducted using SPPS (solid-phase peptide synthesis) approach: All solid-phase peptide synthesis (SPPS) was performed in syringes fitted with internal frits and removable caps on the needle end. The resin remains stuck in the syringe during washing and reagent switching steps. The peptide on the Wang resin **28** was swelled in 1 mL DCM for 30 min and then the solvent was removed. 5.3 mg of **21** (4.7 µmol) was dissolved in 1 mL DCM, treated with 0.9 µL (5.2 µmol) of N,N-diisopropylethylamine (DIPEA) and was added to the pre-swollen **28** (21.8 mg, loading capacity: 0.055 mmol/g). The reaction mixture was then agitated for 2 h at 40°C. The resin was washed thoroughly with DCM (2 × 1 mL), MeOH (2 × 1 mL) and Et₂O (2 × 1 mL). Then, the resin was dried in an oven at 60 °C for 15 min. To cleave the synthesized conjugate **29** from the resin and remove the protecting groups, a cleavage cocktail was added to the resin. This mixture consisted of 95% trifluoracetic acid (TFA), 2.5% H₂O, and 2.5% triisopropylsilane (TIPS). The resin was then agitated for 2 h at room temperature (RT). The cleavage cocktail was collected, and the resin was washed with minimal amounts of DMF (∼ 1 mL). The filtrate was concentrated under a stream of nitrogen and the residue was dissolved in CH₃CN/H₂O (1:4, v/v). The pH of the solution was adjusted between 7 - 8 with 10% ammonia. Finally, 10% (v/v) DMSO was added to the final solution (1 mL per mg) of crude **30** dissolved. The reaction mixture was stirred for 72 h and lyophilized. The crude was purified by semi-preparative HPLC using Gemini C18 (250 x 10mm, 5µ, 110 Å, Phenomenex), A: H2O, 0.1 % (TFA), B: CH₃CN, 0.1 % (TFA), gradient 30 - 65 % B in 25 min, flow rate: 3 mL/min, t_{R} = 17.1 min. The fractions were combined and lyophilized to yield the product **A4** as TFA salt: 2.8 mg (1.26 µmol, 27%)
**A4**·TFA: ESI-MS (pos. CH₃CN/H₂O) m/z: [M+H+Fe³⁺]²⁺ calc.: 1078.36, exp.: 1078.26; [M+H+Fe³⁺]³⁺calc.: 718.90, exp.: 718.99.

### Example 6

### Synthesis of [¹⁷⁷Lu]Lu-A1 and [¹⁷⁷Lu]Lu-A2

To prove the high stability of the complexes according to the invention and the ability of fast radiolabeling compounds **A1** and **A2** according to the invention have been reacted with [¹⁷⁷Lu]LuCl₃ to the complexes [¹⁷⁷Lu]Lu-**A1** and [¹⁷⁷Lu]Lu-**A2**, respectively.

Different concentrations of [¹⁷⁷Lu]Lu-**A1** and [¹⁷⁷Lu]Lu-**A2** were prepared by adding 20 µL (to reach final concentration of 10⁻⁴ M or 10⁻⁶ M) or 2 µL (to reach final concentration of 10⁻⁵ M or 10⁻⁷ M) of **A1** or **A2** (10⁻³ M or 10⁻⁵ stock solution in ddH₂O) to [¹⁷⁷Lu]LuCl₃ (∼5 MBq) diluted in 0.15 M ammonium acetate buffer (0.2 mL, pH 6 or pH 7) at 40 °C. The formation of the radiolutetium complex was verified by radio-TLC and radio-HPLC. A radiochemical yield >98% was achieved for [¹⁷⁷Lu]Lu-**A1** and [¹⁷⁷Lu]Lu-**A2** (up to 10⁻⁶M) at 40°C and pH 6 within 1h.

### Radio-TLC

The degree of radiolabeling was assessed by radio-TLC analysis using system 1: Al₂O₃ (neutral) TLC plates (Merck) and 1:1 (v/v) 1M NH₄OAc/MeOH as eluent system, and system 2: iTLC-SA and an aqueous solution of 0.05 M Na-EDTA (pH 7). Plates were scanned using a BAS-1800II system (Raytest).

### System 1

*R*_{f} value ([¹⁷⁷Lu]Lu-**A1**): 0.85
*R*_{f} value ([¹⁷⁷Lu]Lu-**A2**): 0.85
*R*_{f} value ([¹⁷⁷Lu]Lu³⁺): 0

### System 2

*R*_{f} value ([¹⁷⁷Lu]Lu-**A1**): 0
*R*_{f} value ([¹⁷⁷Lu]Lu-**A2**): 0
*R*_{f} value ([¹⁷⁷Lu]Lu³⁺): 0.9

### Radio-HPLC

The radiochemical purity and yield was monitored by radio-HPLC of [¹⁷⁷Lu]Lu-**A1** and [¹⁷⁷Lu]Lu-**A2** was performed on a Knauer Smartline System, consisting of a Smartline 1000 pump, a K-2501 UV detector, a Raytest Gabi Star activity detector, Chromgate 2.8 software, and a Smartline 5000 manager using a Phenomenex Aqua C18 column (4.6 mm x 250mm, 125 Å, 5 µm). The flow rate was 1 mL/min and the injection volume 10 µL. The solvents used were H₂O + 0.1% TFA (A) and CH₃CN + 0.1% TFA (B). The gradient applied was as follows: 20 min 0% B to 100 % B.
t_{R} = 4.3 min of [¹⁷⁷Lu]Lu³⁺
t_{R} = 16.3 min of [¹⁷⁷Lu]Lu-**A1**
t_{R} = 11.5 min of [¹⁷⁷Lu]Lu-**A2**

### Example 7

### Determination of Partition Coefficients for [¹⁷⁷Lu]Lu-A1 and [¹⁷⁷Lu]Lu-A2

Partition Experiments (log *D*_{O/W)} have been carried out to determine the distribution ratios of [¹⁷⁷Lu]Lu-**A1** or [¹⁷⁷Lu]Lu-**A2** in a two-phase system consisting of n-octanol and water at different pH values. The results are shown in table 3. Two replicates have been made.

**Table 3. Partition Coefficients (log D_{O/W}) of the [¹⁷⁷Lu]Lu-bispidine at different pH**

| | pH | | |
|---|---|---|---|
| | 7.2 | 7.4 | 7.6 |
| [¹⁷⁷Lu]Lu-**A1** | 0.77 ± 0.01 | 0.80 ± 0.01 | 0.80 ± 0.00 |
| [¹⁷⁷Lu]Lu-**A2** | -3.02 ± 0.01 | -3.01 ± 0.01 | -2.98 ± 0.01 |

The determination of distribution ratio log *D*_{*o*/*w*} at 25 ± 1°C followed the standard method used for copper-64. Information about the lipophilicity of the ¹⁷⁷Lu-labeled bispidine ligand **A1** or **A2** was obtained using water/1-octanol mixtures. An aqueous ammonium acetate buffer solution (0.15 M, 380 µL, pH 6) containing **A1** or **A2** (1 mM, 50 µL) and LuCl₃ (100 µM, 50 µL in 0.01 M HCl) spiked with [¹⁷⁷Lu]LuCl₃ (3-4 MBq , 20 µL) was prepared. Full complexation was checked by radio-HPLC and radio-TLC, which gave no evidence of free ¹⁷⁷Lu³⁺ in the aqueous phase. Then, 50 µL of this solution was added to 450 µL of 0.05 M 4-(2-hydroxyethyl)-1-piperazine ethanesulfonic acid (HEPES)-NaOH buffer (pH of 7.2, 7.4, 7.6). The distribution experiments were carried out at room temperature in microcentrifuge tubes (2 cm³) with mechanical shaking for 30 min. The phase ratio V_{(1-octanol)}:V_{(aq)} was 1:1 (0.5 mL each). All samples were centrifuged and the phases then separated. The lutetium complex concentration in both phases was determined radiometrically using γ-radiation [¹⁷⁷Lu, NaI(Tl) scintillation counter, Hidex AMG, automatic gamma counter].

### Example 8

### Stability of [¹⁷⁷Lu]Lu-A1 and [¹⁷⁷Lu]Lu-A2 in the presence of human serum

The stability of the complexes in the presence of human serum (Sigma, product number: H6914, batch number: SLBS2266V) was investigated by radio-SEC. To 10⁻⁴ M solution of **A1** or **A2** in 0.15 M NH₄OAc-buffer pH 6, total volume 0.2 mL) was added 100 MBq [¹⁷⁷Lu]LuCl₃ (in 0.01 M HCl). The reaction mixture was incubated for 5 min at 40°C. After complete complexation (radio-TLC), 100 µL of the [¹⁷⁷Lu]Lu-**A1** or [¹⁷⁷Lu]Lu-**A2** solution, respectively, was transferred to 250 µL of human serum and 50 µL of 1 M HEPES buffer (pH 7.4) was added. The mixture was incubated at 37 °C for 7 d. Time points taken for analysis were 1 h, 1 d, 3 d and 7 d using radio-TLC (system 2).

**Table 4: Stability of [¹⁷⁷Lu]Lu-A1 and [¹⁷⁷Lu]Lu-A2**

| complex | monitored by | intact complex [%]* dependent on time | | | |
|---|---|---|---|---|---|
| | | **1h** | **1d** | **3d** | **7d** |
| [¹⁷⁷Lu]Lu-**A1** | radio- TLC | 94 | 94 | 83 | 76 |
| [¹⁷⁷Lu]Lu-**A2** | radio- TLC | 97 | 96 | 96 | 94 |

| | | | | | |
|---|---|---|---|---|---|
| "absolute value which is not bound/dissociated to human serum | | | | | |

### Example 9

### Synthesis of [²²⁵Ac]Ac-A1 and [²²⁵Ac]Ac-A2

To prove the high stability of the complexes according to the invention and the ability of fast radiolabeling compounds **A1** and **A2** according to the invention have been reacted with [²²⁵Ac]AcNO₃ to the complexes [²²⁵Ac]Ac-**A1** and [²²⁵Ac]Ac-**A2**, respectively.

Different concentrations of [²²⁵Ac]Ac-**A1** and [²²⁵Ac]Ac-**A2** were prepared by adding 20 µL (to reach final concentration of 10⁻⁴ M or 10⁻⁶ M) or 2 µL (to reach final concentration of 10⁻⁵ M or 10⁻⁷ M) of **A1** or **A2** (10⁻³ M or 10⁻⁵ stock solution in ddH₂O) to [²²⁵Ac]AcNO₃ (40-100 kBq) diluted in 0.15 M ammonium acetate buffer (0.2 mL, pH 6 or pH 7) at 40 and 80°C. The formation of the radio complex was verified by radio-TLC. A radiochemical yield >98% was achieved up to 10⁻⁶ M at pH 6 for [²²⁵Ac]Ac-**A2** at 40°C and for [²²⁵Ac]Ac-**A1** at 80°C up to 10⁻⁵ M within 5 min.

### System 2

*R*_{f} value ([²²⁵Ac]Ac-**A1**): 0
*R*_{f} value ([²²⁵Ac]Ac-**A2**): 0
*R*_{f} value (([²²⁵Ac]Ac³⁺): 0.85

### Example 19

### Stability of [²²⁵Ac]Ac-A1 and [²²⁵Ac]Ac-A2 in the presence of human serum

The stability of the complexes in the presence of human serum (Sigma, product number: H6914, batch number: SLBS2266V) was investigated by radio-TLC (system 2). To 10⁻⁴ M solution of **A1** or **A2** in 0.15 M NH₄OAc-buffer pH 6, total volume 0.2 mL) was added 600 kBq [²²⁵Ac]AcNO₃ (in 0.01 M HCl). The reaction mixture was incubated for 60 min at 40°C (for **A2**) or 80°C (for **A1**). After complete complexation (radio-TLC), 100 µL of the [²²⁵Ac]Ac-**A1** or [²²⁵Ac]Ac-**A2**, respectively, (∼300 kBq) solution was transferred to 250 µL of human serum and 50 µL of 1 M HEPES buffer (pH 7.4) was added. The mixture was incubated at 37 °C for 10 d. Time points taken for analysis were 1 h, 1 d, 3 d, 7 d and 10 d using radio-TLC (system 2).

**Table 5: Stability [%] of [²²⁵Ac]Ac-A1 and [²²⁵Ac]Ac-A2**

| complex | monitored by | intact complex [%]* dependent on time | | | | |
|---|---|---|---|---|---|---|
| | | 1h | 1d | 3d | 7d | 10d |
| [²²⁵Ac]Ac-**A1** | radio- TLC | 98 | 97 | 83 | 73 | 66 |
| [²²⁵Ac]Ac-**A2** | radio- TLC | 99 | 98 | 95 | 94 | 93 |

| | | | | | | |
|---|---|---|---|---|---|---|
| *absolute value which is not bound/dissociated to human serum | | | | | | |

## Claims

1. A compound of general formula I 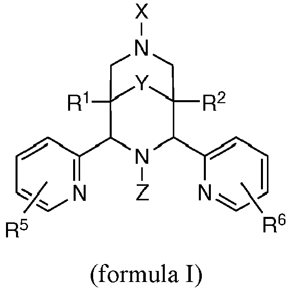 in which
X is selected from a first group or a second group or third group, wherein the first group consists of 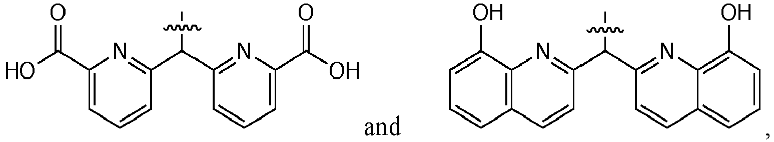 the second group consists of 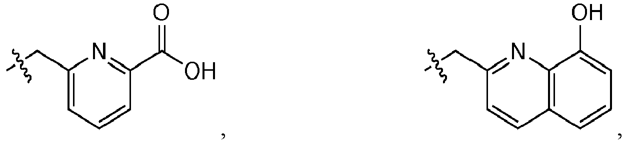 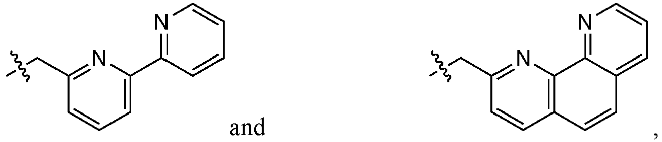 and the third group consists of 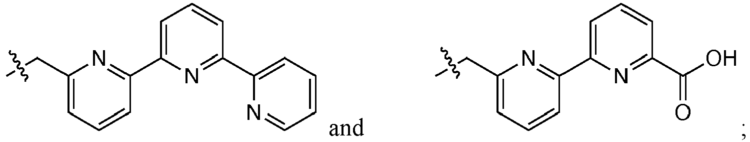
Y is a group of general formula II or of general formula III 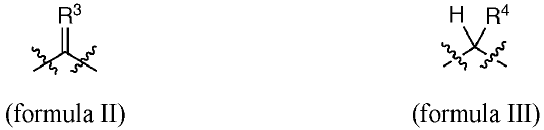
if X is selected from the first group, Z is selected from a first group consisting of 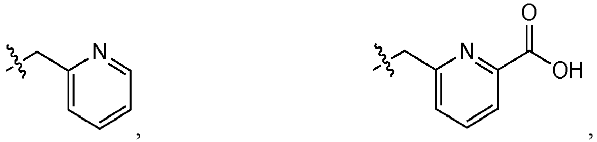 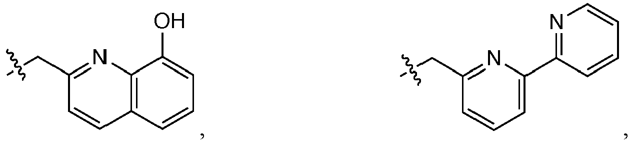 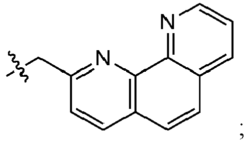
if X is selected from the second group, Z is selected from a second group consisting of 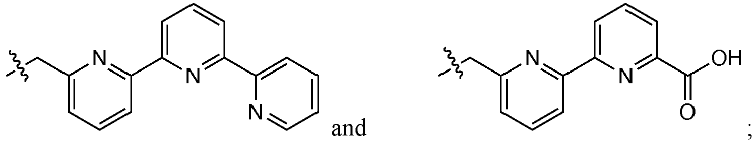
if X is selected from the third group, Z is selected from a third group consisting of 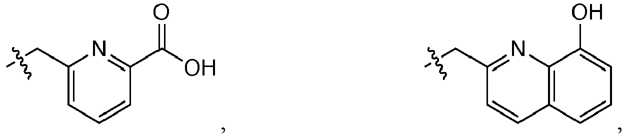 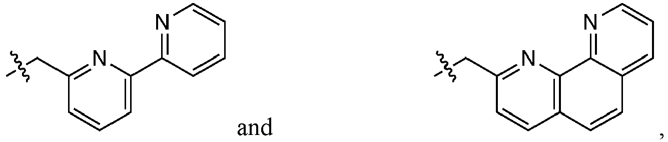
R¹ and R² independently are selected from the group consisting of a substituted or unsubstituted C₁-C₆ alkyl group, a substituted or unsubstituted C₁-C6 heteroalkyl group, -C(O)-O-R^{a}, -C(O)-NR^{b}R^{c}, -C(O)-C(R^{a})₂-NR^{b}R^{c}, and-C(O)-NR^{b}-(CH₂)ₙ-C(O)-O-R^{a}, wherein R^{a}, R^{b} and R^{c} each independently are selected from the group consisting of hydrogen, a substituted or unsubstituted C₁-C₆ alkyl group, a substituted or unsubstituted C₁-C₆ heteroalkyl group, a substituted or unsubstituted aryl group, a group -A-L and a group L and n is an integer from 1 to 10;
R³ is selected from the group consisting of oxygen, sulfur, =NR^{d}, and =CHR^{d}, wherein R^{d} is selected from the group consisting of hydrogen, a substituted or unsubstituted C₁-C₆ alkyl group, a substituted or unsubstituted C₁-C₆ heteroalkyl group, a substituted or unsubstituted aryl group, -O-R^{e}, -C(O)-O-R^{e}, -C(O)-NR^{f}R^{g}, a group -A-L and a group L, R^{e} is hydrogen or a substituted or unsubstituted C₁-C₆ alkyl group, and R^{f} and R^{g} each independently are hydrogen or a substituted or unsubstituted C₁-C₆ alkyl group; and
R⁴ is selected from the group consisting of -OR^{h}, -SR^{h}, -NHR^{h} and -CH₂R^{h}, wherein R^{h} is selected from the group consisting of hydrogen, a substituted or unsubstituted C₁-C₆ alkyl group, a substituted or unsubstituted C₁-C₆ alkenyl group, a substituted or unsubstituted C₁-C₆ alkynyl group, a substituted or unsubstituted C₁-C₆ heteroalkyl group, a substituted or unsubstituted aryl group, -C(O)-(CH₂)ₘ-R^{k}, -C(O)-(CH₂)ₘ-NR^{m}Rⁿ, a group -A-L and a group L, R^{k} is selected from the group consisting of a substituted or unsubstituted C₁-C₆ alkyl group, a substituted or unsubstituted C₁-C₆ heteroalkyl group, a substituted or unsubstituted aryl group, and a substituted or unsubstituted carboxy group, R^{m} and Rⁿ each independently are hydrogen or a substituted or unsubstituted C₁-C₆ alkyl group, and m is 0 or an integer from 1 to 10;
R⁵ and R⁶ independently are selected from the group consisting of hydrogen, chloro, bromo, iodo, and O-R^{o}, wherein R^{o} is a substituted or unsubstituted C₁-C₆ alkyl group;
A is a linker group and
L is an amino acid residue or a peptide.

2. The compound according to claim 1, **characterized in that** R¹ and R² independently are -C(O)-O-R^{a} or -(CH)ₙ-C(O)-O-R^{a}, wherein R^{a} has the meaning given in claim 1.

3. The compound according to claim 1 or claim 2, **characterized in that** R³ is selected from the group consisting of oxygen and =NR^{d}, wherein R^{d} has the meaning given in claim 1.

4. The compound according to any of the preceding claims, **characterized in that** R⁴ is a hydroxy group or -OR^{h}, wherein R^{h} has the meaning given in claim 1.

5. The compound according to any of the preceding claims, **characterized in that** X is a group of formula 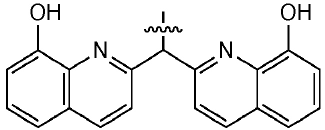 and Z is a group of formula

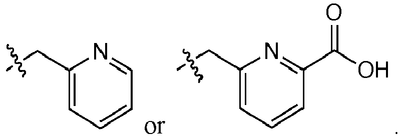

6. The compound according to any of claims 1 to 4, **characterized in that** X is a group of formula 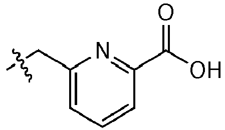 and Z is a group of formula

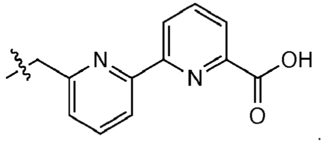

7. The compound according to any of claims 1 to 5, **characterized in that** it is a compound of formula A1, A2, or A3:

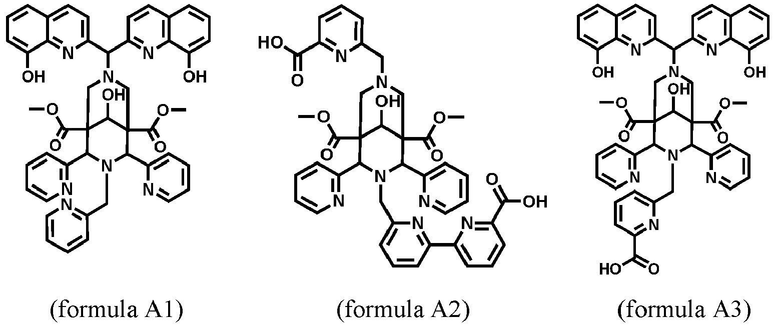

8. The compound according to any of claims 1 to 6, **characterized in that** the group -A-L is a group of formula L1

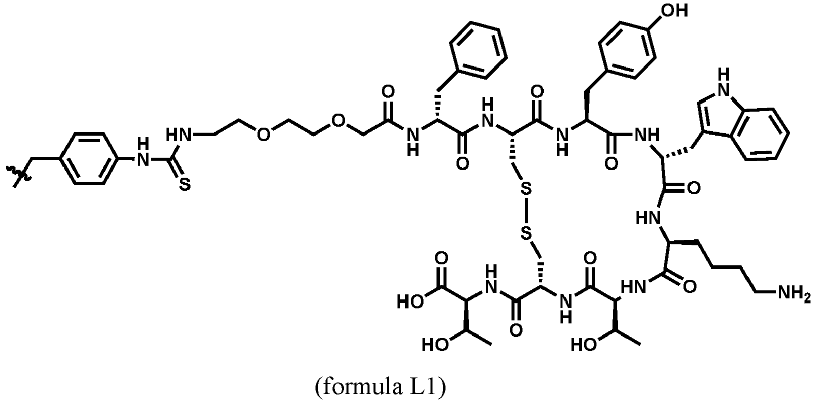

9. The compound according to any of the preceding claims, **characterized in that** it is a compound of formula A4: 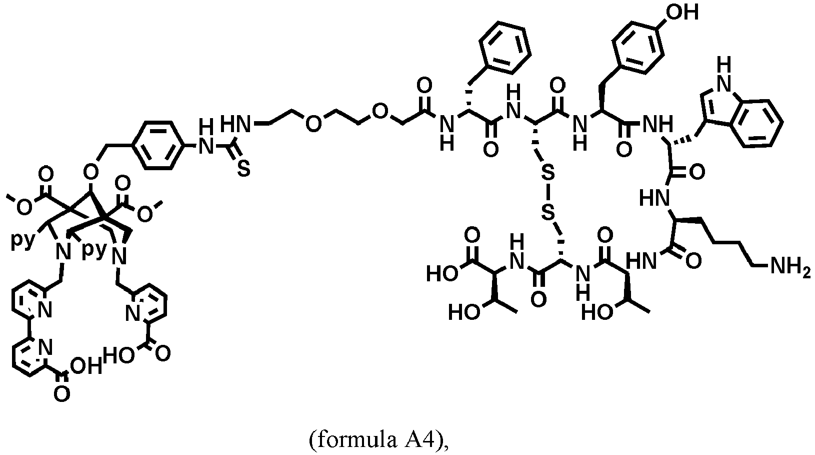 in which the designation "py" indicates a pyridyl group.

10. Use of a compound according to any of claims 1 to 9 for the complexation of lutetium ions or actinium ions.

11. The use according to claim 10, **characterized in that** the compound is used for the complexation of a metal ion.

12. A method for the complexation of lutetium ions or actinium ions, wherein a compound according to any of claims 1 to 9 is contacted with a metal ion at a reaction temperature in the range of from 20 to 50°C.

13. The method according to claim 12, **characterized in that** the compound according to claims 1 to 9 is reacted with a metal salt at ambient pressure.

14. A complex of a compound according to any of claims 1 to 7 as ligands and a metal ion.

15. The complex according to claim 14, **characterized in that** the ligand is a nonadentate or decadentate ligand.
